# EUROPEAN PATENT APPLICATION

(11) **EP 4 650 453 A1**
(43) Date of publication of application: **19.11.2025**
(21) Application number: 24741377.6
(22) Date of filing: 12.01.2024
(51) Int. Cl.: C12N 15/85

(54) **ANTIBIOTIC-FREE MINIPLASMID, AND PREPARATION METHOD THEREFOR AND USE THEREOF**

(30) Priority: 13.01.2023 CN 202310072956
(71) Applicant: Maxirna (Shanghai) Pharmaceutical Co., Ltd., Songjiang District Shanghai 201601 (CN); Maxirna (Zhejiang) Technology Co., Ltd., Shaoxing, Zhejiang 312467 (CN); Shanghai Cell Therapy Group Co., Ltd, Jiading District Anting Town Shanghai 201805 (CN)
(72) Inventor: ZHANG, Pingjing, Shanghai 201805 (CN); WANG, Xiaoxue, Shanghai 201805 (CN); LIU, Tao, Shanghai 201805 (CN); YAO, Jianyun, Shanghai 201805 (CN); NI, Songwei, Shanghai 201805 (CN); WANG, Pengxia, Shanghai 201805 (CN); SUN, Yan, Shanghai 201805 (CN); QIAN, Qijun, Shanghai 201805 (CN)
(74) Representative: Nederlandsch Octrooibureau
(86) International application number: PCT/CN2024/072052
(87) International publication number: WO 2024/149383

(57) **Abstract**

Provided is an antibiotic-free miniplasmid comprising a nucleotide sequence encoding an antitoxin protein and a replicon. The antibiotic-free miniplasmid has a small backbone controlled within 1000 bp, redundant and useless fragments are reduced and the miniplasmid does not contain a resistance gene expression cassette, so that the utilization rate of a target sequence in the plasmid is increased, and the production burden is reduced. The miniplasmid has higher efficiency and safety in genetic engineering such as plasmid DNA-mediated non-viral vector delivery, gene therapy, vaccine immunization, virus production, antibody production, etc. Further provided is a system for producing a plasmid on the basis of a toxin-antitoxin system. The bacterial host cell contains a gene expression cassette capable of inducing expression of a toxin protein, and the antibiotic-free miniplasmid contains a gene expression cassette for expressing the antitoxin protein, which is used for efficiently maintaining replication and amplification of the antibiotic-free miniplasmid.

## Description

The present application claims priority to Chinese patent application 2023100729561, filed on January 13, 2023. The content of the Chinese patent application is incorporated herein by reference in its entirety.

### TECHNICAL FIELD

The present application belongs to the technical field of biological engineering, relates to an antibiotic resistance gene-free miniplasmid and use thereof, and further relates to a system for efficiently producing the antibiotic resistance gene-free miniplasmid and a genetically engineered bacterium thereof.

### BACKGROUND

Gene therapy receives more and more attention. Currently, over thousands of clinical trials have gained global approval. The field of "non-viral" gene therapy, based on plasmid DNA *in vivo* or in cells, has steadily increased, with increasing requirements on the demands and varieties of plasmids.

The primary concern is the issue of antibiotic safety of the plasmids. In plasmid production, when a plasmid is conveyed to progeny bacteria or cells, a bacterial system can ensure stable replication and production only when each daughter cell comprises at least one copy of the plasmid. However, the plasmids actually produce an additional metabolic load on production strains and strongly influence their growth rates. Plasmid-free cells have a growth advantage and can enable the entire population to overgrow. In order to solve the above problems, the production of plasmid DNA needs to be able to exert a selection pressure advantageous for selecting bacteria comprising a target plasmid after transformation and during amplification of bacteria. Generally, genes conferring antibiotic resistance are commonly used as plasmid selective markers and bacteria need to be cultured in a medium containing an antibiotic. However, the use of antibiotic resistance genes as selective markers for plasmid production poses safety issues often addressed by regulatory agencies. For example, horizontal gene transfer to the bacteria of a patient cannot be eliminated, and therefore, residual antibiotics in the final product may cause allergic reactions in susceptible individuals. In view of this, a new generation of plasmid backbones without antibiotic resistance markers has emerged to improve the safety of non-viral gene therapy trials. A second concern is that the sizes of the plasmids and the bacterially derived DNA fragments affect the functions of the plasmids, for example, smaller plasmids are more prone to enter the nucleus, thereby increasing the efficiency of plasmid DNA integration; fewer bacterially derived CpG DNA motifs result in lower immunogenicity, thereby favoring transgene expression.

Existing antibiotic resistance gene-free plasmid maintaining strategies mainly include complementation of auxotrophic strains, toxin-antitoxin systems, operon-repressor regulation, RNA regulation, etc. The strategies further comprise forming antibiotic resistance gene-free minicircle or microline plasmids by recombinase enzyme digestion of antibiotic resistance genes and replicon DNA sequences in a mother vector.

A toxin-antitoxin (TA) system was first discovered in 1983 on low-copy plasmids. The system has important significance for increasing the stability and spread of the plasmids in populations, and is once considered to be a plasmid addiction system. The TA system is widely found in chromosomes and plasmids of prokaryotes and archaebacteria. The system consists of 2 co-expression genes encoding a stable toxin protein and a readily degradable antitoxin, respectively. The toxin normally exerts a toxic effect to inhibit bacterial growth, while the antitoxin neutralizes toxicity. Some TA systems are separated and modified into killing systems and have become a method for plasmid selection by replacing antibiotics. The principle is post-segregational killing (PSK) of toxin and antitoxin of the TA system. Namely, after plasmids in daughter cells are lost, antitoxin molecules with a short half-life period are quickly degraded, free toxin proteins are released to exert molecular toxicity, and finally the daughter cells with lost plasmids die, thereby ensuring the stability of the plasmids in populations.

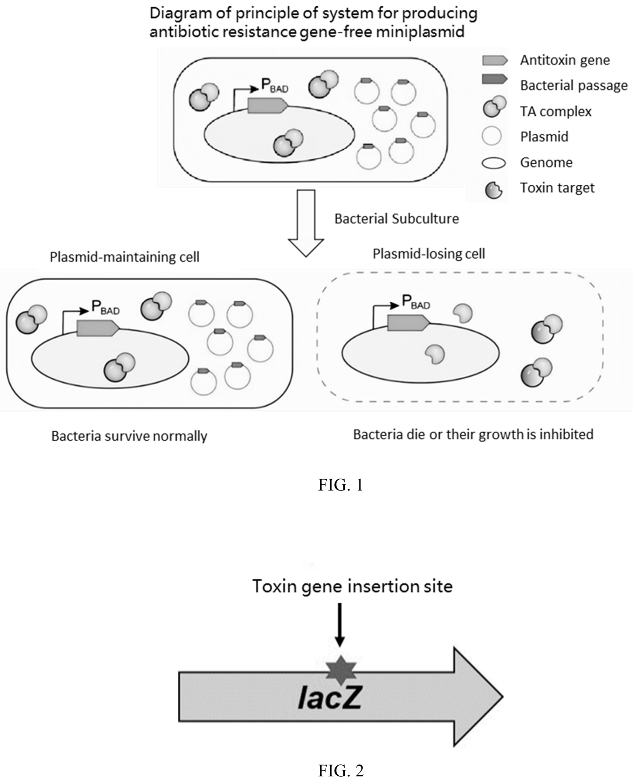

For example, an *E.coli* CcdB protein derived from *Escherichia coli* is an inhibitor of an essential gyrase for growth, and is toxic to *Enterobacteriaceae,* but not toxic to eukaryotic cells. An *E.coli* CcdA gene encodes an antidote that interacts with toxin. When an *E.coli* CcdB toxin gene is integrated into the bacterial chromosome, the CcdA gene is inserted into the expression plasmid. In the case of plasmid deletion or loss, toxin is produced and induces cell death. The separation of the two components ensures efficient killing of plasmid-free cells, thereby increasing plasmid-containing bacteria and plasmid production. FIG. 1 shows a diagram of a working principle of a screening system for antibiotic resistance gene-free miniplasmids.

Many types of bacterial toxin-antitoxin systems in nature have been reported, including CcdB (CcdA antitoxin), Kis (Kid antitoxin), Phd (Doc antitoxin), RelB (RelE antitoxin), PasB (or PasC) (PasA antitoxin), MazF (MazE antitoxin), etc. Besides, each type of toxin and antitoxin differs in different bacterial species. Since the lethality of toxins from different species, the sizes of antitoxin genes, and the efficiency of toxin neutralization are different, it is well known to those skilled in the art that not all types of toxin and antitoxin systems can be adapted to an antibiotic resistance gene-free plasmid production system.

### SUMMARY

In order to solve the technical problems, the present application provides an antibiotic resistance gene-free miniplasmid, a system for producing the antibiotic resistance gene-free miniplasmid by using a toxin-antitoxin system, and use of the antibiotic resistance gene-free miniplasmid. The antibiotic resistance gene-free miniplasmid of the present application avoids use of an antibiotic resistance gene and can be stably replicated and efficiently produced, has a smaller plasmid backbone, and can be used in the non-viral vector delivery, gene therapy, DNA vaccine immunity, virus production, antibody production and other genetic engineering. The antibiotic resistance gene-free miniplasmid of the present application is also called "Tini plasmid".

A first aspect of the present application provides an antibiotic resistance gene-free miniplasmid, comprising a nucleotide sequence encoding an antitoxin protein, and a replicon, wherein an amino acid sequence of the antitoxin protein comprises the following sequence: (1) an amino acid sequence shown in SEQ ID NO: 1, or an amino acid sequence having one or more mutations of E24D, I36V and V43I compared with SEQ ID NO: 1; or (2) an amino acid sequence shown in SEQ ID NO: 9, or an amino acid sequence having one or more mutations of T6I, T43A, K47E, A50S, E51D, G52A and N54K compared with SEQ ID NO: 9; and a length of the replicon is ≤ 800 bp, preferably ≤ 600 bp or ≤ 300 bp.

Wherein, the amino acid sequence of the antitoxin protein is shown in SEQ ID NO: 1, SEQ ID NO: 2, SEQ ID NO: 3, SEQ ID NO: 9, SEQ ID NO: 10, SEQ ID NO: 11 or SEQ ID NO: 12.

Preferably, the replicon is selected from ColE1, ColE2, pMB1, pSC101, RSF, R6K, pUC57, RK2, and p15A, preferably R6K or pUC57.
wherein, an antitoxin protein is derived from a toxin-antitoxin system provided by the present application, the toxin-antitoxin system includes a nucleotide sequence encoding a toxin protein and a nucleotide sequence encoding an antitoxin protein, and the toxin protein and the antitoxin protein are selected from any one of the following groups:
(1) an amino acid sequence of the toxin protein is shown in SEQ ID NO: 4, and an amino acid sequence of the antitoxin protein is shown in SEQ ID NO: 3; and
(2) an amino acid sequence of the toxin protein is shown in SEQ ID NO: 13, and an amino acid sequence of the antitoxin protein is shown in SEQ ID NO: 12.

In a preferred embodiment, the toxin-antitoxin system is selected from any one of the following groups:
(1) the nucleotide sequence encoding a toxin protein is shown in SEQ ID NO: 6; the nucleotide sequence encoding an antitoxin protein is shown in SEQ ID NO: 5, or the nucleotide sequence shown in SEQ ID NO: 5 not comprising a CpG motif; and
(2) the nucleotide sequence encoding a toxin protein is shown in SEQ ID NO: 15; the nucleotide sequence encoding an antitoxin protein is shown in SEQ ID NO: 14, or the nucleotide sequence shown in SEQ ID NO: 14 not comprising a CpG motif.

Preferably, a length of a plasmid backbone of the antibiotic resistance gene-free miniplasmid is ≤ 1,000 bp, preferably ≤ 900 bp, ≤ 800 bp or ≤ 600 bp.

The nucleotide sequence encoding an antitoxin protein does not comprise a CpG motif and preferably, the nucleotide sequence encoding an antitoxin protein is shown in SEQ ID NO: 7 or SEQ ID NO: 16.

Preferably, a nucleotide sequence of a replicon does not comprise a CpG motif, for example, an R6K replicon does not comprise a CpG motif shown in SEQ ID NO: 20.

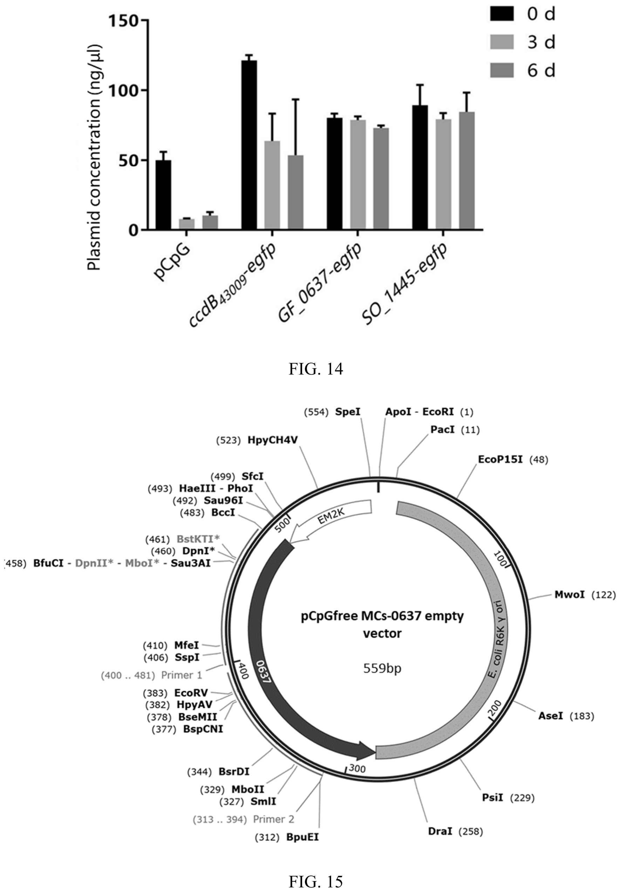

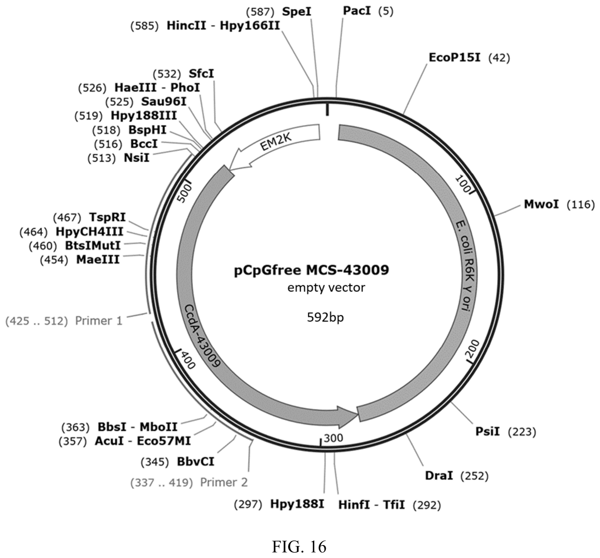

In a preferred embodiment, the antibiotic resistance gene-free miniplasmid does not compris a gene of interest. The nucleotide sequence is shown in SEQ ID NO: 8 or 17. The map structures of the plasmids are shown in FIGS. 15-16.

Preferably, the antibiotic resistance gene-free miniplasmid comprises a structured DNA sequence selected from a polyA repeat, an SV40 origin of replication, a viral LTR, a lentiviral LTR, a retroviral LTR, a transposon IR/DR, an AAV ITR, a transposon ITR, a CMV enhancer, and an SV40 enhancer.

Preferably, the antibiotic resistance gene-free miniplasmid is selected from a viral vector, a lentiviral vector, a retroviral vector, an AAV vector, an Ad vector, a Sleeping Beauty transposon vector, a PiggyBac transposon vector, a ZB transposon vector, a PS transposon vector, a Tol2 transposon vector, and a polyA-containing mRNA vector.

Preferably, the antibiotic resistance gene-free miniplasmid comprises an exogenous gene, and preferably, the exogenous gene encodes a chimeric antigen receptor and/or encodes an antibody.

A second aspect of the present application provides a recombinant host cell comprising the antibiotic resistance gene-free miniplasmid of the present application.

A third aspect of the present application provides a method for preparing a cell, including introducing the antibiotic resistance gene-free miniplasmid of the present application into a cell.

Preferably, the cell is from an animal, for example, a vertebrate or an invertebrate, preferably a mammal, further preferably a human; and/or the cell is an immune cell, preferably a T cell. Preferably, the introducing comprises transfecting the cell by electroporation, microinjection, calcium phosphate precipitation, a cationic polymer, a dendrimer, a liposome, microparticle bombardment, fugene, direct acoustic wave loading, cell extrusion, optical transfection, protoplast fusion, impalefection, magnetic transfection, nucleofaction, or any combination thereof, preferably by electroporation.

A fourth aspect of the present application provides a pharmaceutical composition, including the antibiotic resistance gene-free miniplasmid or the recombinant host cell of the present application, and an optionally pharmaceutically acceptable excipient.

A fifth aspect of the present application provides a system for producing a plasmid, comprising a host cell and a plasmid, wherein the host cell comprises a nucleotide sequence encoding a toxin protein, and the plasmid comprises a nucleotide sequence encoding an antitoxin protein;
an amino acid sequence of the toxin protein is shown in SEQ ID NO: 4, and an amino acid sequence of the antitoxin protein comprises: an amino acid sequence shown in SEQ ID NO: 1, or an amino acid sequence having one or more mutations of E24D, I36V and V43I compared with SEQ ID NO: 1; or
an amino acid sequence of the toxin protein is shown in SEQ ID NO: 13, and an amino acid sequence of the antitoxin protein comprises: an amino acid sequence shown in SEQ ID NO: 9, or an amino acid sequence having one or more mutations of T6I, T43A, K47E, A50S, E51D, G52A and N54K compared with SEQ ID NO: 9.

Preferably, the amino acid sequence of the toxin protein is shown in SEQ ID NO: 4, and the amino acid sequence of the antitoxin protein is shown in SEQ ID NO: 1, SEQ ID NO: 2, or SEQ ID NO: 3; or the amino acid sequence of the antitoxin protein is shown in SEQ ID NO: 13, and the amino acid sequence of the antitoxin protein is shown in SEQ ID NO: 9, SEQ ID NO: 10, SEQ ID NO: 11 or SEQ ID NO: 12.

Preferably, the host cell is a Gram-negative bacterium, preferably Escherichia coli, such as an Escherichia coli GT115 strain, a Top10 strain, a DH5a strain, or a BL21 (DE3) strain. Preferably, a bacterial host cell is an *Escherichia coli* containing a Pai protein necessary for an R6K replicon.

Preferably, the host cell comprises a gene expression cassette for inducibly expressing the toxin, the gene expression cassette for the toxin protein comprises an operon or a promoter for inducibly expressing the toxin protein, and preferably, the operon or the promoter is selected from a Lac lactose operon, an L-arabinose inducible pBAD promoter, an L-rhamnose inducible rhapBAD promoter, or a λPL/PR-clts857 thermosensitive promoter.

Preferably, the plasmid comprises a replicon. Preferably, the replicon is selected from ColE1, ColE2, pMB1, pSC101, RSF, R6K, pUC57, RK2, and p15A, preferably R6K or pUC57.
the plasmid does not compris an antibiotic resistance gene, and/or a length of the plasmid backbone is ≤ 1,000 bp, preferably ≤ 900 bp, ≤ 800 bp or ≤ 600 bp.

In a preferred embodiment, when the replicon in the plasmid backbone of the antibiotic resistance gene-free miniplasmid is pUC57, ColE1, and pMB1, the length of the plasmid backbone is ≤ 1,000 bp.

In a preferred embodiment, when the replicon in the plasmid backbone of the antibiotic resistance gene-free miniplasmid is an R6K replicon, the length of the plasmid backbone is ≤ 600 bp.

Preferably, the replicon and/or the nucleotide sequence encoding an antitoxin protein do not compris a CpG motif after optimization.

A sixth aspect of the present application provides a method for producing a plasmid by using the system of the present application, including the following steps:
(1) transforming the plasmid containing the nucleotide sequence encoding an antitoxin protein into the host cell comprising the nucleotide sequence encoding a toxin protein;
(2) inducibly expressing the toxin protein during continuous culture of a bacterial host cell and expressing the antitoxin protein; and
(3) obtaining a plasmid, wherein preferably, the plasmid is the antibiotic resistance gene-free miniplasmid of the present application.

The present application provides a method for producing a target recombinant peptide, polypeptide or protein by using the system according to any one of claims 15-21, wherein the plasmid further comprises a gene expression cassette encoding the target recombinant peptide, polypeptide or protein, and the method comprises culturing the system and recovering the recombinant peptide, polypeptide or protein of interest.

In a preferred embodiment, arabinose is added during the culture to induce the expression of a gene encoding a toxin protein.

A seventh aspect of the present application provides a genetically engineered bacterium, wherein a cell of the genetically engineered bacterium comprises a nucleotide sequence encoding a toxin protein, an amino acid sequence of the toxin protein is shown in SEQ ID NO: 4 or 13, and preferably, the nucleotide sequence encoding a toxin protein is within a genome of the genetically engineered bacterium.

Preferably, the genetically engineered bacterium further comprises a plasmid, and the plasmid comprises a nucleotide sequence encoding an antitoxin protein. and the plasmid comprises a nucleotide sequence encoding an antitoxin protein;
an amino acid sequence of the toxin protein is shown in SEQ ID NO: 4, and an amino acid sequence of the antitoxin protein comprises: an amino acid sequence shown in SEQ ID NO: 1, or an amino acid sequence having one or more mutations of E24D, I36V and V43I compared with SEQ ID NO: 1; or
an amino acid sequence of the toxin protein is shown in SEQ ID NO: 13, and an amino acid sequence of the antitoxin protein comprises: an amino acid sequence shown in SEQ ID NO: 9, or an amino acid sequence having one or more mutations of T6I, T43A, K47E, A50S, E51D, G52A and N54K compared with SEQ ID NO: 9.

Preferably, the plasmid is the antibiotic resistance gene-free miniplasmid of the present application.

Preferably, the genetically engineered bacterium is a Gram-negative bacterium, preferably *Escherichia coli,* such as an *Escherichia coli* GT115 strain, a Top10 strain, a DH5a strain, or a BL21 (DE3) strain.

In some embodiments, the bacterial host cell is an *Escherichia coli* comprising a Pai protein necessary for an R6K replicon, and the Pai protein is expressed by a *pir* gene.

The present application further provides a kit, including the antibiotic resistance gene-free miniplasmid, the recombinant host cell, the system or the genetically engineered bacterium of the present application.

The present application further provides use of the antibiotic resistance gene-free miniplasmid, the recombinant host cell, the system, the genetically engineered bacterium, or the kit of the present application in preparing a drug for gene therapy, a drug for cell therapy and a DNA vaccine, and producing a virus or an antibody.

The present application further provides use of the antibiotic resistance gene-free miniplasmid, the recombinant host cell, the system, the genetically engineered bacterium, or the kit in maintaining stability of a plasmid in a bacterial host cell.

The present application further provides use of the toxin-antitoxin system in maintaining stability of a plasmid in a bacterial host cell. The plasmid may comprise an antibiotic resistance gene, and the toxin-antitoxin system can further improve the stability of the plasmid. The plasmid may not comprise an antibiotic resistance gene, and the toxin-antitoxin system alone can also maintain the stability of the plasmid.

The present application further provides a method for screening a toxin-antitoxin system, including:
(I) inserting a gene expression cassette for inducibly expressing a toxin protein into a bacterial host genome to induce expression of an antitoxin protein; and
(II) inserting a gene expression cassette for inducibly expressing a toxin protein into a bacterial host genome and introducing a plasmid comprising an antitoxin gene into bacteria to induce expression of an antitoxin protein.

The gene for expressing a toxin protein and the gene for expressing an antitoxin belong to the same group of toxin-antitoxin system.

When the bacterial host cell in (I) exhibits a suicide effect and the bacterial host cell in (II) grows normally and the plasmid is stably passaged, an effective toxin-antitoxin system is obtained by screening.

The present application further provides a method of inserting a nucleotide sequence encoding a toxin protein in the toxin-antitoxin system according to the present application into a bacterial genome, including: cloning a gene encoding a toxin protein to a plasmid inducibly expressed by arabinose and inserting the gene into a *lacZ* gene site in a bacterial genome through a homologous recombination method.

The length of the antibiotic resistance gene-free miniplasmid of the present application is smaller, redundant useless fragments are reduced, and meanwhile, the miniplasmid does not comprise a resistance expression cassette, thereby improving the utilization rate of a sequence of interest in the plasmid and reducing the production burden. Besides, a backbone of the antibiotic resistance gene-free miniplasmid is controlled within 1,000 bp, the low toxicity of the plasmid is ensured, and meanwhile, the expression of the plasmid is improved. Compared with common plasmids, the antibiotic resistance gene-free miniplasmid of the present application can improve the expression of exogenous genes, has low immunogenicity through CpG optimization, is free of antibiotic resistance genes, and improves the safety in the use process.

### BRIEF DESCRIPTION OF THE DRAWINGS

FIG. 1 shows a diagram of a working principle of a screening system for antibiotic resistance gene-free miniplasmids.
FIG. 2 shows a schematic diagram of inserting toxin into a GT115 genome.
FIG. 3 shows a schematic diagram of inserting a toxin gene into pBAD33-oriT.
FIG. 4 shows the result of PCR verification of positive transformants.
FIG. 5 shows a schematic diagram of a fused PCR fragment for inserting into the GT115 genome.
FIG. 6 shows PCR identification of verification primers of the GT115 genome;
FIG. 7 shows the growth curve measurement of strains GT115::Ara promoter (control), GT115::SO_1444, GT115::ccdB (43009), GT115::ccdB (*E.coli*) and GT115::0636; A: after overnight activation of the strains, the initial OD is adjusted to 0.1 and the growth of the different strains at each time point of 2 h, 4 h, 6 h and 8 h are determined; and 0.2% glucose is added to inhibit the activity of an arabinose promoter in the process of strain culture; and B: the number of colonies gradiently diluted of each strain at time point of 8 h.

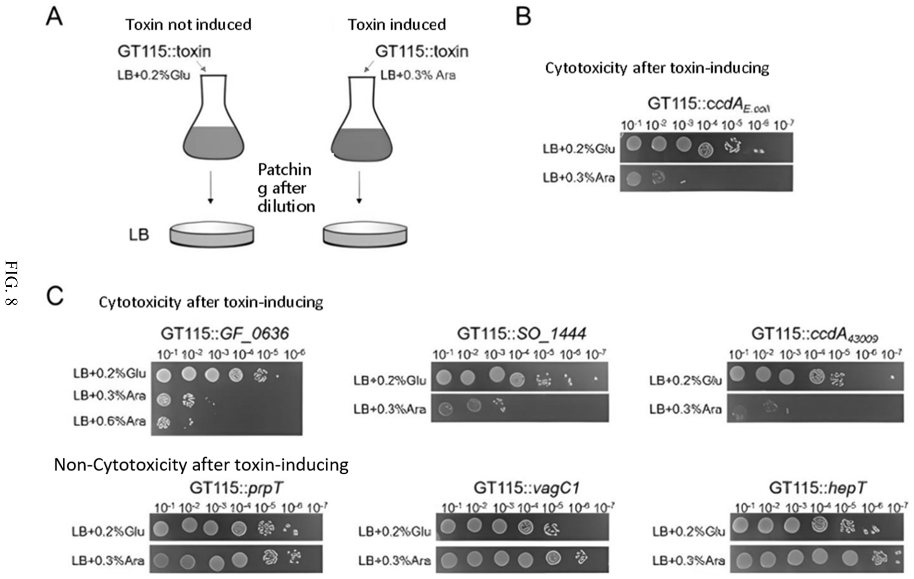

FIG. 8 shows toxicity detection of 6 pairs of candidate TA systems after toxin expression; A: experimental flow chart; after the strains are cultured overnight, 1% of the strains are respectively transferred into an LB medium containing 0.2% Glu (glucose) and 0.3% Ara (arabinose), and after 2 h of induction, the strains are respectively patched correspondingly on an LB plate and the survival rate of the bacteria is calculated; B: toxicity detection of toxin ccdA*_{E.coli}* of CcdA/CcdB (*E.coli*) as a control; and C: cytotoxicity after toxin expression in three pairs of TA systems, namely, SO_1444/SO_1445, CcdA/CcdB (43009) and GF_0636/GF_0637, and non-cytotoxicity after toxin expression in three pairs of TA systems, namely, VagC1/VagD1, PrpT/PrpA and HepT/MntA.
FIG. 9 shows screening of antitoxin-inserted antibiotic resistance gene-free plasmid pCpG-antitoxin by using toxicity of toxin; A: schematic diagram of construction of antitoxin-inserted antibiotic resistance gene-free plasmid pCpG-antitoxin; and B: PCR verification diagram of transforming corresponding antitoxin-inserted antibiotic resistance gene-free plasmid pCpG-antitoxin in a toxin-expressing host.
FIG. 10 shows the comparison of the effect of the construction of the TA systems on strain growth;
FIG. 11 shows the insertion of a target gene *egfp* by using the antibiotic resistance gene-free miniplasmid pCpG-antitoxin; A: schematic diagram of inserting the target gene *egfp* by using the antibiotic resistance gene-free miniplasmid pCpG-antitoxin; and B: PCR verification of egfp-inserted recombinant plasmids and efficiency detection.
FIG. 12 shows plasmid detection after 3 days of continuous transfer culture of antibiotic resistance gene-free miniplasmids, wherein pCpG: pCpGfree-MCS, *ccdA₄₃₀₀₉:* pCpG-*ccdA₄₃₀₀₉, SO_1445:pCpG-SO_1445, GF_0637:*pCpG-*GF_0637, ccdA_{E.coli}:* CpG*-ccdA_{E.coli}.*
FIG. 13 shows the plasmid gel graph at 0 d, 3 d and 6 d after continuous passage of egfp-inserted antibiotic resistance gene-free miniplasmids (second batch of experiments).
FIG. 14 shows the plasmid concentrations at 0 d, 3 d and 6 d after continuous passage of the egfp-inserted antibiotic resistance gene-free miniplasmids.
FIG. 15 shows a map of a pCpGfree MCS-0637 empty miniplasmid, with a corresponding nucleotide sequence shown in SEQ ID NO: 8.
FIG. 16 shows a map of a pCpGfree MCS-43009 empty miniplasmid, with a corresponding nucleotide sequence shown in SEQ ID NO: 17.
FIG. 17 shows a map of a pCpGfree MCS-1445 empty miniplasmid, with a corresponding nucleotide sequence shown in SEQ ID NO: 18.
FIG 18 shows a map of a pCpGfree MCS-ccdA *E.coli* empty miniplasmid, with a corresponding nucleotide sequence shown in SEQ ID NO: 19.
FIG. 19 shows a map of a pCpG-ccdA₄₃₀₀₉-EGFP plasmid.
FIG. 20 shows a map of a pCpG-GF-0637-EGFP plasmid.
FIG. 21 shows the effectiveness of truncated antitoxin proteins in the antibiotic resistance gene-free miniplasmids.
FIG. 22 shows flow cytometry of Example 8 and analyzes expression quantity and expression continuity of different plasmids.
FIG. 23 shows fluorescence photography of Example 8 and analyzes expression quantity and expression continuity of different plasmids.
FIG. 24 shows the positive rate of integration of a plasmid-loaded eGFP expression cassette into Jurkat cells by a ZB transposase.
FIG. 25 shows the positive rate of integration of the plasmid-loaded eGFP expression cassette into PBMCs by the ZB transposase.
FIG. 26 shows the nucleic acid electrophoresis of a pucTA-6.5A-MDR1-EGFP-90A plasmid extracted from different clones in Example 10.
FIG. 27 shows the nucleic acid electrophoresis of the pucTA-6.5A-MDR1-EGFP-90A plasmid extracted from different generations in Example 10.

### DETAILED DESCRIPTION OF EMBODIMENTS

The term "toxin-antitoxin" (TA) refers to a group of a toxin protein and an antitoxin protein, wherein the toxin protein is toxic to a host cell and the antitoxin protein can neutralize the toxicity of the toxin protein to the host cell. When the cell has a chromosome or a plasmid-encoded antitoxin gene, the antitoxin protein can be expressed continuously to neutralize the toxin protein and maintain the survival of the cell. If the plasmid carrying the antitoxin gene is lost from the cell, the synthesized toxin protein will be left longer than the antitoxin protein and can kill the cell or inhibit cell growth. Herein, the terms "toxin protein" and "toxin" are used interchangeably and refer to proteins in the toxin-antitoxin system that are capable of killing cells or inhibiting the cell growth; and the terms "antitoxin protein" and "antitoxin" are used interchangeably and refer to proteins in the toxin-antitoxin system that form a complex with the toxin protein to neutralize the toxicity of the toxin protein.

The term "codon optimization" refers to a technique for improving and maximizing protein expression in a living organism by increasing the translation efficiency of a gene of interest by transforming/replacing a DNA sequence of a nucleotide of one species with a nucleotide sequence of another species. The codon optimization involves replacing a wild-type DNA sequence and a rare codon with a more highly expressed species sequence and a frequently occurring codon without altering the protein.

A CpG motif refers to an immunostimulatory CpG oligonucleotide, i.e., a short single-stranded synthetic nucleic acid molecule, that contains a deoxynucleotide cytosine triphosphate ("C") and a deoxynucleoside guanine triphosphate ("G"). "p" refers to a phosphodiester or phosphorothioate linkage between consecutive nucleotides. Unmethylated CpG motifs are considered pathogen associated molecular patterns (PAMPs), because they are abundant in microbial genomes but rare in vertebrate genomes. The present application removes the CpG motif in a nucleotide sequence encoding an antiviral protein through codon optimization.

Although the toxin-antitoxin systems are widely present in the chromosomes and plasmids of prokaryotes and archaebacteria, not all toxin-antitoxin systems can be used to maintain plasmid stability. For example, a part of toxins have low lethality and cannot be used for efficient screening; genes of a part of the toxin-antitoxin systems are over-sized and can cause the obtained plasmid backbone to be large; and the replication and expression efficiencies of a part of the toxins-antitoxins are too low, affecting use of the plasmid. The present application screens out a proper toxin-antitoxin system based on an antibiotic resistance gene-free miniplasmid.

The present application provides the toxin-antitoxin system suitable for the antibiotic resistance gene-free miniplasmid. The toxin-antitoxin system is derived from a marine strain *Roseivirga spongicola_*GF047 and includes nucleotide sequences encoding a toxin protein GF_0636 and an antitoxin protein GF_0637; or derived from a marine strain *Vibrio* sp. 43009 and includes nucleotide sequences encoding a toxin protein CcdB (43009) or an antitoxin protein CcdA

(43009). Compared with a toxin-antitoxin system (comprising nucleotide sequences encoding a toxin protein SO_1444 (1444 for short) and an antitoxin protein SO_1445 (1445 for short)) derived from a marine strain *Shewanella oneidensis* MR-1, the toxin-antitoxin system provided by the present application has a shorter length. Therefore, the antibiotic resistance gene-free miniplasmid is smaller, has lower toxicity, and has higher transposition efficiency when used as a vector of a transposon system.

In some embodiments, an amino acid sequence of the toxin protein GF_0636 of the marine strain is shown in SEQ ID NO: 4, and an amino acid sequence of the antitoxin protein GF_0637 of the marine strain is shown in SEQ ID NO: 3.

In some embodiments, the nucleotide sequence encoding the toxin protein GF_0636 is shown in SEQ ID NO: 6, and the nucleotide sequence encoding the antitoxin protein GF_0637 is shown in SEQ ID NO: 5, or the sequence shown in SEQ ID NO: 5 not comprising a CpG motif.

In some embodiments, the nucleotide sequence encoding the antitoxin protein GF_0637 does not comprise a CpG motif after optimization and is shown in SEQ ID NO: 7.

In some embodiments, an amino acid sequence of the toxin CcdB (43009) derived from the marine strain *Vibrio* sp. 43009 is shown in SEQ ID NO: 13, and an amino acid sequence of the antitoxin CcdA (43009) is shown in SEQ ID NO: 12.

In some embodiments, the nucleotide sequence encoding the toxin CcdB (43009) is shown in SEQ ID NO: 15, and the nucleotide sequence encoding the antitoxin CcdA (43009) is shown in SEQ ID NO: 14, or the sequence shown in SEQ ID NO: 14 not comprising a CpG motif.

In some embodiments, the nucleotide sequence encoding the antitoxin CcdA (43009) does not comprise a CpG motif after optimization and is shown in SEQ ID NO: 16.

An amino acid sequence of the toxin SO_1444 of the marine strain *Shewanella oneidensis* MR-1 is shown in SEQ ID NO: 22, and an amino acid sequence of the antitoxin SO_1445 is shown in SEQ ID NO: 21; and the nucleotide sequence encoding the toxin SO_1444 is shown in SEQ ID NO: 24, and the nucleotide sequence encoding the antitoxin SO_1445 is shown in SEQ ID NO: 23.

The term "antibiotic resistance gene-free plasmid" refers to a plasmid that does not comprise an antibiotic resistance gene. The term "miniplasmid" or "small plasmid" refers to a small (less than 4 kb) circular plasmid derivative that has been isolated from all prokaryotic vector parts (i.e., they do not comprise bacterial DNA sequences) and used as a transgenic vector for genetic modification of mammalian cells. The miniplasmid and use thereof are described in: Minicircle and Miniplasmid DNA Vectors: The Future of Nonviral and Viral Gene Transfer, Dr. Martin Schleef, May 2013, Wiley-Blackwell, page 258. The term "antibiotic resistance gene-free miniplasmid" refers to a miniplasmid that does not comprise an antibiotic resistance gene.

The term "plasmid backbone" refers to a DNA sequence at least including elements necessary for autonomous replication of a plasmid in a bacterial host.

The term "replicon" refers to a DNA sequence that enables bacteria, plasmids or vectors to autonomously replicate and maintain a normal copy number, and is also referred to as "origin of replication" in some literature.

The toxin-antitoxin system of the present application can maintain a stable presence of a plasmid in a host cell, and therefore, the plasmid may include other target genes of interest which are stably expressed in the host cell, for example, for the production of polypeptides. Similarly, the plasmid may include exogenous genes, but does not express the exogenous genes in the bacterial host cells. By stable replication and passage of the plasmid, the antibiotic resistance gene-free miniplasmid not comprising antibiotic resistance genes and comprising exogenous genes is directly obtained, and can be used for gene editing, gene therapy, etc.

The present application provides an antibiotic resistance gene-free miniplasmid, comprising a nucleotide sequence encoding an antitoxin protein, and a replicon, wherein an amino acid sequence of the antitoxin protein comprises the following sequence: (1) an amino acid sequence shown in SEQ ID NO: 1, or an amino acid sequence having one or more mutations of E24D, 136V and V43I compared with SEQ ID NO: 1; or (2) an amino acid sequence shown in SEQ ID NO: 9, or an amino acid sequence having one or more mutations of T6I, T43A, K47E, A50S, E51D, G52A and N54K compared with SEQ ID NO: 9; and a length of the replicon is ≤ 800 bp, preferably ≤ 600 bp or ≤ 300 bp.

A mutation of E24D refers to the mutation of the 24th amino acid from E to D. The same applies to other mutations.

An amino acid sequence shown in SEQ ID NO: 1 is a truncated sequence of the antitoxin protein GF_0637 and is the shortest sequence for realizing the antitoxin function, such that the plasmid can be further shortened. The antitoxin protein GF_0637 is derived from the marine strain *Roseivirga spongicola*_GF047 and generates natural mutation during the strain culture process. The known natural mutation includes E24D, I36V, and V43I. The mutated antitoxin protein can still realize the same antitoxin function. Therefore, the antitoxin protein GF_0637 and a truncated sequence thereof may include one or more mutations of E24D, I36V, and V43I. Similarly, the amino acid sequence shown in SEQ ID NO: 9 is a truncated sequence of the antitoxin protein CcdA (43009). The known natural mutations include T6I, T43A, K47E, A50S, E51D, G52A, and N54K. Therefore, the antitoxin protein and a truncated sequence thereof may include one or more mutations of T6I, T43A, K47E, A50S, E51D, G52A, and N54K.

In some embodiments, wherein the amino acid sequence of the antitoxin protein is shown in SEQ ID NO: 1, SEQ ID NO: 2, SEQ ID NO: 3, SEQ ID NO: 9, SEQ ID NO: 10, SEQ ID NO: 11 or SEQ ID NO: 12.

the replicon is selected from ColE1, ColE2, pMB1, pSC101, RSF, R6K, pUC57, RK2, and p 15A. In consideration of plasmid copy number, plasmid toxicity and plasmid backbone size, the replicon is preferably R6K or pUC57. The antibiotic resistance gene-free miniplasmid may be amplified and replicated along with the culture of the bacterial host cell, and the copy number of the antibiotic resistance gene-free miniplasmid in the bacterial host cell is increased, such that a large amount of the antibiotic resistance gene-free miniplasmid may be obtained in a short time.

The existing miniplasmid has a plasmid backbone generally less than 4,000 bp. Since the plasmid generally comprises an antibiotic resistance gene with a length of 810 bp, the existing miniplasmid has the plasmid backbone length generally greater than 1,200 bp. The antibiotic resistance gene-free miniplasmid of the present application uses the antitoxin protein gene to replace the antibiotic resistance gene, thereby greatly reducing the length of the plasmid backbone. The length of the nucleotide sequence of the antitoxin protein of the present application is ≤ 300 bp. The length of the plasmid backbone of the antibiotic resistance gene-free miniplasmid of the present application is ≤ 1,000 bp, for example, ≤ 900 bp, ≤ 800 bp or ≤ 600 bp.

In a preferred embodiment, when the replicon in the plasmid backbone of the antibiotic resistance gene-free miniplasmid is pUC57, ColE1, and pMB1, the length of the plasmid backbone is ≤ 1,000 bp.

In a preferred embodiment, when the replicon in the plasmid backbone of the antibiotic resistance gene-free miniplasmid is an R6K replicon, the length of the plasmid backbone is ≤ 600 bp.

The plasmid comprises the nucleotide sequence encoding the antitoxin protein. When used in mammals such as humans, the CpG motif in the nucleotide sequence encoding the antitoxin protein may be removed through codon optimization, thereby reducing immunogenicity. The nucleotide sequence encoding the toxin protein and the nucleotide sequence encoding the antitoxin protein are not on the same plasmid. Therefore, the CpG motif in the nucleotide sequence encoding the toxin protein may not be removed.

The replicon and/or the nucleotide sequence encoding the antitoxin protein do not comprise the CpG motif after the optimization. In some embodiments, the nucleotide sequence encoding the antitoxin protein is shown in SEQ ID NO: 7 or SEQ ID NO: 16. In some embodiments, the R6K replicon does not comprise a CpG motif and is shown in SEQ ID NO: 20.

A gene expression cassette for expressing the antitoxin protein of the antibiotic resistance gene-free miniplasmid comprises a promoter, preferably a bacterial promoter, such as but not limited to, EM2K, J23119, and Tac, such that the antitoxin protein is continuously expressed in the bacterial host cell.

The antibiotic resistance gene-free miniplasmid of the present application may not comprise a gene of interest. An empty plasmid is directly obtained by using a system for producing the antibiotic resistance gene-free miniplasmid of the present application. A nucleotide sequence is shown in SEQ ID NO: 8 or 17. The map structures of the plasmids are shown in FIGS. 15-16.

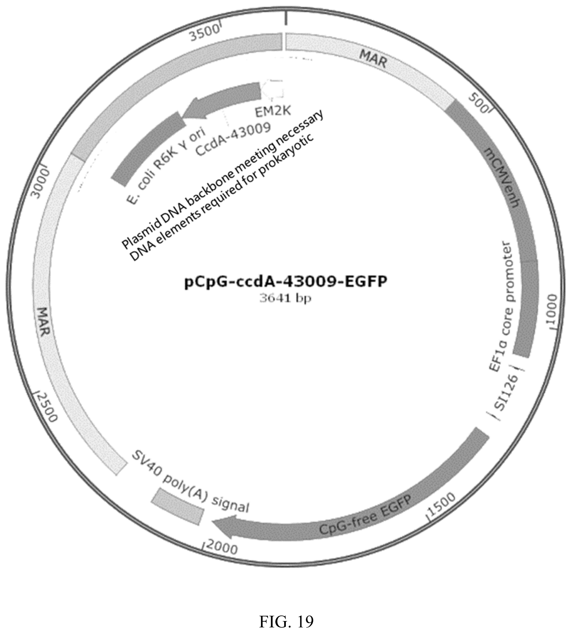

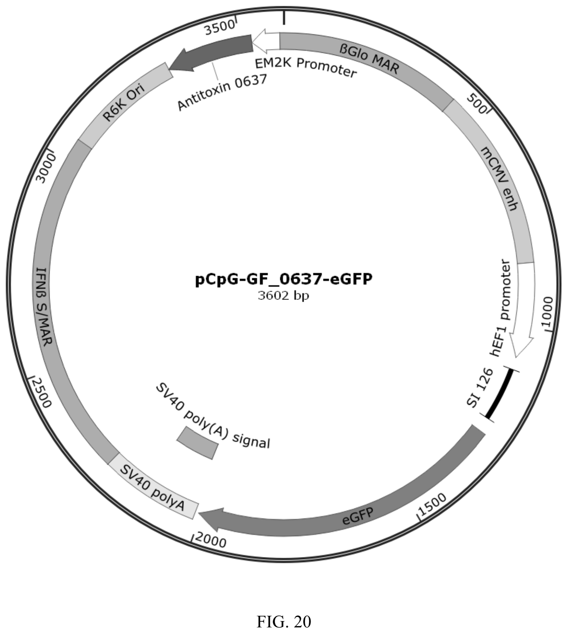

The antibiotic resistance gene-free miniplasmid of the present application may comprise a gene of interest. After the antibiotic resistance gene-free miniplasmid of the present application loads the gene of interest, since the miniplasmid has a smaller plasmid length, the miniplasmid has a larger potential in non-viral vector delivery. The gene of interest may be an antibody gene, a chimeric antigen receptor gene, a gene-editing enzyme gene, an antigen gene, a viral gene, etc. In the examples of the present application, to verify the effect of the antibiotic resistance gene-free miniplasmid, the gene of interest may further be a reporter gene, for example, an EGFP reporter gene. The map structures of typical antibiotic resistance gene-free miniplasmids are shown in FIGS. 19-20.

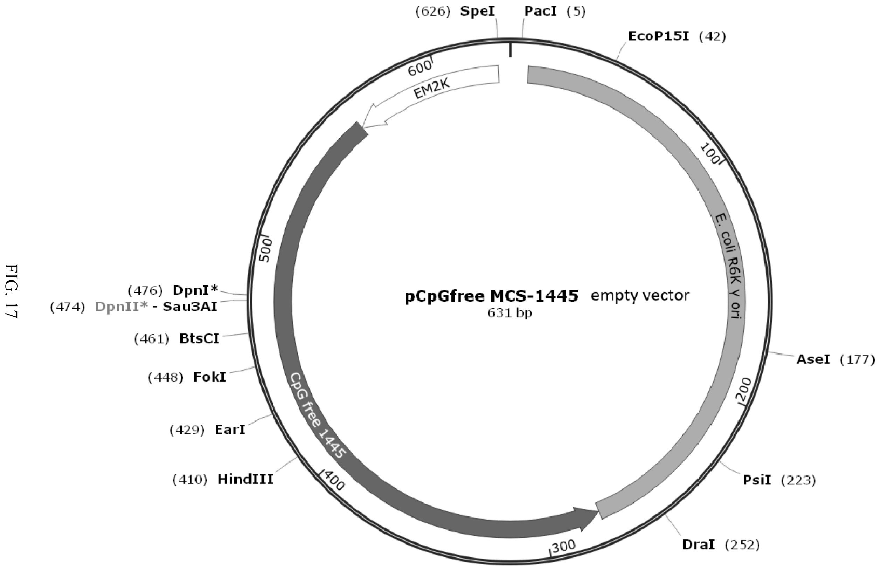

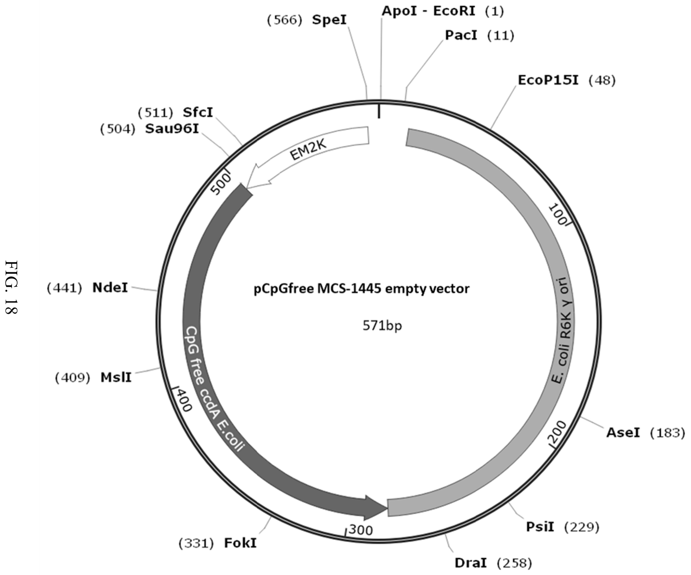

A map of a pCpGfree MCS-1445 empty miniplasmid is shown in FIG. 17 and a map of a pCpGfree MCS-ccdA *E.coli* empty miniplasmid is shown in FIG. 18.

In some embodiments, the antibiotic resistance gene-free miniplasmid comprises a structured DNA sequence selected from a polyA repeat, an SV40 origin of replication, a viral LTR, a lentiviral LTR, a retroviral LTR, a transposon IR/DR, an AAV ITR, a transposon ITR, a CMV enhancer, and an SV40 enhancer.

In some embodiments, the antibiotic resistance gene-free miniplasmid is selected from a Sleeping Beauty transposon vector, a PiggyBac transposon vector, a ZB transposon vector, a PS transposon vector, a Tol2 transposon vector, and a polyA-containing mRNA vector.

A ZB transposon vector may refer to CN105018523B and a PS transposon vector may refer to CN110257425B.

In some embodiments, the antibiotic resistance gene-free miniplasmid comprises an exogenous gene. Preferably, the exogenous gene encodes a chimeric antigen receptor and/or encodes an antibody. For example, the antibiotic resistance gene-free miniplasmid may comprises an exogenous gene encoding a chimeric antigen receptor and is used for preparing an immune cell of the chimeric antigen receptor, such as CAR-T. The antibiotic resistance gene-free miniplasmid may comprise an exogenous gene encoding an antibody, for example may comprise a PD-1 antibody gene. The antibiotic resistance gene-free miniplasmid may further simultaneously comprises the exogenous genes encoding the chimeric antigen receptor and encoding the antibody. The exogenous genes may be in the same antibiotic resistance gene-free miniplasmid or may be used in combination in different antibiotic resistance gene-free miniplasmids.

The present application further provides a recombinant host cell comprising the antibiotic resistance gene-free miniplasmid of the present application. A method for preparing the recombinant host cell is introducing the antibiotic resistance gene-free miniplasmid of the present application into a cell.

Preferably, the cell is from an animal, for example, a vertebrate or an invertebrate, preferably a mammal, further preferably a human; and/or the cell is an immune cell, preferably a T cell.

Preferably, the introducing comprises transfecting the cell by electroporation, microinjection, calcium phosphate precipitation, a cationic polymer, a dendrimer, a liposome, microparticle bombardment, fugene, direct acoustic wave loading, cell extrusion, optical transfection, protoplast fusion, impalefection, magnetic transfection, nucleofaction, or any combination thereof, preferably by electroporation.

The present application further provides a pharmaceutical composition including the antibiotic resistance gene-free miniplasmid or the recombinant host cell of the present application, and an optionally pharmaceutically acceptable excipient.

The present application further provides a system for producing a plasmid, comprising a host cell and a plasmid, wherein the host cell comprises a nucleotide sequence encoding a toxin protein, and the plasmid comprises a nucleotide sequence encoding an antitoxin protein;
an amino acid sequence of the toxin protein is shown in SEQ ID NO: 4, and an amino acid sequence of the antitoxin protein comprises: an amino acid sequence shown in SEQ ID NO: 1, or an amino acid sequence having one or more mutations of E24D, I36V and V43I compared with SEQ ID NO: 1; or
an amino acid sequence of the toxin protein is shown in SEQ ID NO: 13, and an amino acid sequence of the antitoxin protein comprises: an amino acid sequence shown in SEQ ID NO: 9, or an amino acid sequence having one or more mutations of T6I, T43A, K47E, A50S, E51D, G52A and N54K compared with SEQ ID NO: 9.

In some embodiments, the amino acid sequence of the toxin protein is shown in SEQ ID NO: 4, and the amino acid sequence of the antitoxin protein is shown in SEQ ID NO: 1, SEQ ID NO: 2, or SEQ ID NO: 3; or the amino acid sequence of the antitoxin protein is shown in SEQ ID NO: 13, and the amino acid sequence of the antitoxin protein is shown in SEQ ID NO: 9, SEQ ID NO: 10, SEQ ID NO: 11 or SEQ ID NO: 12.

The principle of the system for producing a plasmid of the present application is to maintain the stability of the plasmid in the bacterial host cell by using a toxin-antitoxin system. Along with the culture and proliferation of the bacterial host cell, the plasmid is replicated and amplified so as to be efficiently produced. The system for producing an antibiotic resistance gene-free miniplasmid of the present application includes a bacterial host cell and a plasmid. The plasmid may be in the bacterial host cell and may be produced by directly culturing the bacterial host cell. Or the plasmid and the bacterial host cell are stored separately. When ready for use, the bacterial host cell is transfected with the plasmid.

In some embodiments, the plasmid includes an antibiotic resistance gene capable of maintaining the stability of the plasmid. The system includes the toxin-antitoxin system such that the stability of the plasmid can be further improved. Therefore, the system of the present application may be used to produce the plasmid including the antibiotic resistance gene.

In some embodiments, the plasmid does not comprise the antibiotic resistance gene. The stability of the plasmid is dependent on the toxin-antitoxin system. At this time, the system of the present application is used to produce the antibiotic resistance gene-free plasmid. If only the production of the plasmid is taken into consideration, the size of the antibiotic resistance gene-free plasmid is not particularly limited. However, if use of the antibiotic resistance gene-free plasmid is considered, through selection of suitable replicons, a smaller antibiotic resistance gene-free plasmid, for example, the length of the antibiotic resistance gene-free plasmid backbone is ≤ 1,000 bp, preferably ≤ 900 bp, ≤ 800 bp or ≤ 600 bp, may be obtained. Preferably, the antibiotic resistance gene-free plasmid is the antibiotic resistance gene-free miniplasmid of the present application.

In some embodiments, the host cell is a Gram-negative bacterium, preferably *Escherichia coli,* such as an *Escherichia coli* GT115 strain, a Top10 strain, a DH5a strain, or a BL21 (DE3) strain.

In some embodiments, the bacterial host cell is an *Escherichia coli* comprising a Pai protein necessary for an R6K replicon, and the Pai protein is expressed by a pir gene.

In some embodiments, the toxin bacterial host cell comprises a gene expression cassette for inducibly expressing a toxin protein, the toxin protein is expressed under the inducing condition, and the bacterial host cell comprising the plasmid is screened out. In some embodiments, the gene expression cassette for expressing the toxin protein may be inserted into a genome of the bacterial host cell or located in a plasmid. The gene expression cassette for expressing the toxin protein is inserted into the genome of the bacterial host cell and is stably present and expressed in the host cell. Preferably, for example, the gene expression cassette is inserted into a *lacZ* region of a genome of GT115 *Escherichia coli.* The gene expression cassette for expressing the toxin protein may further be located in other plasmids in addition to the plasmid produced. At this time, the other plasmids may further comprise an antibiotic resistance gene, avoiding the loss of the plasmid. Since the other plasmid is not a final product and is not used in gene therapy, it will not cause abuse of the antibiotic resistance gene. The gene expression cassette for inducibly expressing the toxin protein comprises an operon or a promoter. The toxin protein is expressed under the inducing condition. The operon or the promoter is selected from a Lac lactose operon, an L-arabinose inducible pBAD promoter, an L-rhamnose inducible rhapBAD promoter, and a λPL/PR-clts857 thermosensitive promoter.

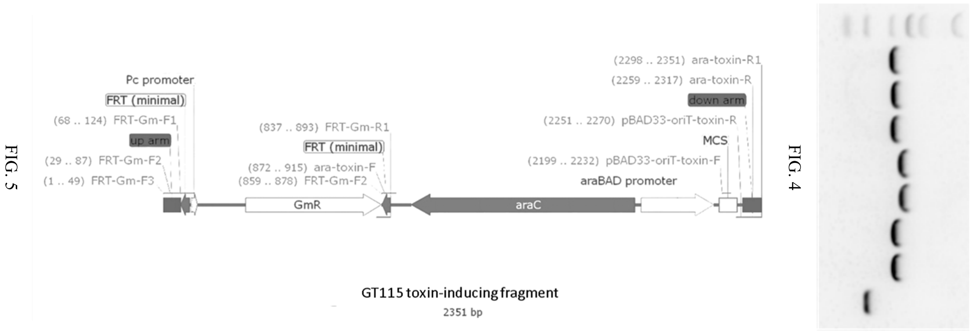

In some embodiments, the promoter is linked to the gene expression cassette of the toxin protein in a manner shown in FIG. 5.

In some embodiments, according to the kinds of the promoters or operons, the culture conditions are adjusted to induce the expression of the toxin protein, e.g., when the L-arabinose inducible pBAD promoter is used, arabinose may be added to induce expression of a gene encoding the toxin protein during the culture.

In some embodiments, the plasmid comprises the gene expression cassette for expressing an antitoxin protein. The plasmid is in the bacterial host cell. An antitoxin gene in the plasmid and a toxin gene in the bacterial genome or other plasmids form a toxin-antitoxin system. The plasmid is matched with the bacterial host cell comprising the antitoxin gene for use. During the culture of the bacterial host cell, the toxin protein and the antitoxin protein are expressed simultaneously. Through the screening effect of the toxin-antitoxin system, the plasmid is stably present in the cultured bacterial host cell.

In some embodiments, the replicon is an R6K replicon whose nucleotide sequence is shown in SEQ ID NO: 20. In some embodiments, the replicon and a sequence of an antitoxin gene expression cassette in the antibiotic resistance gene-free miniplasmid do not comprise a CpG motif after optimization.

Preferably, the sequence of the antitoxin gene expression cassette not comprising the CpG motif is shown in SEQ ID NO: 7 or 16.

The system of the present application may be used for producing a plasmid, for example, by fermenting and culturing the bacterial host cell in the system, the plasmid of the present application is obtained.

The present application further provides a method for producing a plasmid by using the system, including the following steps: (1) transforming the plasmid comprising the nucleotide sequence encoding an antitoxin protein into the host cell comprising the nucleotide sequence encoding a toxin protein; (2) inducibly expressing the toxin protein during culture of a bacterial host cell and expressing the antitoxin protein; and (3) obtaining a plasmid.
preferably, the plasmid is the antibiotic resistance gene-free miniplasmid of the present application.

The conditions of the fermentation and culture may be a conventional antibiotic-free subculture in the art, for example, an LB medium is used for the fermentation and culture. In the culture process, according to the kinds of the promoters or operons, the culture conditions are adjusted to induce the expression of the toxin protein. Since the expression of the toxin protein may cause the death or the growth inhibition of a bacterial host cell, a controllable induced expression is used. The induced expression may be performed at an initial stage of culture or after the bacterial host cell is subcultured for a certain period of time. The expression of the antitoxin protein has little influence on the bacterial host cell and may be continuously expressed in the culture process. After the culture is finished, the bacteria are collected and the plasmid is extracted so as to obtain the antibiotic resistance gene-free miniplasmid.

The present application further provides a method for producing a target recombinant peptide, polypeptide or protein by using the system. The plasmid further comprises a gene expression cassette encoding the target recombinant peptide, polypeptide or protein. The method includes culturing the system and recovering the target recombinant peptide, polypeptide or protein.

The production of plasmids primarily takes into account the copy number of the plasmids, obtains the end product being the plasmids and does not involve expression of recombinant peptides, polypeptides or proteins by the plasmids. Unlike the production of plasmids, the production and expression of recombinant peptides, polypeptides or proteins also involves the copy number of the plasmids and requires the plasmids to express the recombinant peptides, the polypeptides or the proteins, so as to finally obtain the recombinant peptides, the polypeptides or the proteins.

Examples of the peptides, the polypeptides or the proteins produced by the method of the present application are, but are not limited to, enzymes, regulatory proteins, receptors, peptides (e.g., peptide hormones), cytokines, antibodies, nanobodies, membrane proteins or transporters.

In some embodiments, the host cell further comprises a nucleic acid sequence encoding a protein that induces expression of the target peptide, polypeptide or protein, preferably placed in the gene expression cassette for encoding the target recombinant peptide, polypeptide or protein.

The present application provides a genetically engineered bacterium, wherein a cell of the genetically engineered bacterium comprises a nucleotide sequence encoding a toxin protein, an amino acid sequence of the toxin protein is shown in SEQ ID NO: 4 or 13, and preferably, the nucleotide sequence encoding a toxin protein is within a genome of the genetically engineered bacterium.

The genetically engineered bacterium further comprises a plasmid, and the plasmid comprises a nucleotide sequence encoding an antitoxin protein;
an amino acid sequence of the toxin protein is shown in SEQ ID NO: 4, and an amino acid sequence of the antitoxin protein comprises: an amino acid sequence shown in SEQ ID NO: 1, or an amino acid sequence having one or more mutations of E24D, I36V and V43I compared with SEQ ID NO: 1; or
an amino acid sequence of the toxin protein is shown in SEQ ID NO: 13, and an amino acid sequence of the antitoxin protein comprises: an amino acid sequence shown in SEQ ID NO: 9, or an amino acid sequence having one or more mutations of T6I, T43A, K47E, A50S, E51D, G52A and N54K compared with SEQ ID NO: 9.

Preferably, the plasmid is the antibiotic resistance gene-free miniplasmid of the present application.

Preferably, the genetically engineered bacterium is a Gram-negative bacterium, preferably *Escherichia coli,* such as an *Escherichia coli* GT115 strain, a Top10 strain, a DH5a strain, or a BL21 (DE3) strain.

The present application further provides a kit, including the antibiotic resistance gene-free miniplasmid, the recombinant host cell, the system or the genetically engineered bacterium of the present application.

The present application further provides use of the antibiotic resistance gene-free miniplasmid, the recombinant host cell, the system, the genetically engineered bacterium, or the kit of the present application in preparing a drug for gene therapy, a drug for cell therapy and a DNA vaccine, and producing a virus or an antibody.

In some embodiments, the use is selected from any one of (1) to (6) as follows:
(1) use in preparing a drug or a reagent for integrating a target gene expression cassette into a genome of a host cell;
(2) use in preparing a tool for integrating a target gene expression cassette into a genome of a host cell;
(3) use in preparing a transgenic animal and a transgenic cell;
(4) use in preparing a drug or a reagent for genome research, gene therapy, cell therapy or stem cell induction and differentiation after the induction;
(5) use in preparing a tool for genome research, gene therapy, cell therapy or stem cell induction and differentiation after the induction; and
(6) use in preparing a kit, an engineered immune cell or a pharmaceutical composition.

The present application further provides use of the antibiotic resistance gene-free miniplasmid, the recombinant host cell, the system, the genetically engineered bacterium, or the kit in maintaining stability of a plasmid in a bacterial host cell.

The present application further provides use of the toxin-antitoxin system in maintaining stability of a plasmid in a bacterial host cell. The plasmid may comprise an antibiotic resistance gene and the toxin-antitoxin system can further improve the stability of the plasmid. The plasmid may not comprise an antibiotic resistance gene, and the toxin-antitoxin system alone can also maintain the stability of the plasmid.

In some specific embodiments, maintaining the stability of the plasmid may be classified as:
the bacterial plasmid comprises antitoxin protein and antibiotic resistance genes, and the bacterial genome comprises a toxin protein gene;
the bacterial plasmid comprises toxin protein, antitoxin protein and antibiotic resistance genes; and
the bacterial plasmid comprises an antitoxin protein gene, but no antibiotic resistance gene, and the bacterial genome comprises a toxin protein gene.

The present application provides a method for screening a toxin-antitoxin system, including:
(I) inserting a gene expression cassette for inducibly expressing a toxin protein into a bacterial host genome to induce expression of an antitoxin protein; and
(II) inserting a gene expression cassette for inducibly expressing a toxin protein into a bacterial host genome and introducing a plasmid comprising an antitoxin gene into bacteria to induce expression of an antitoxin protein.

The gene for expressing a toxin protein and the gene for expressing an antitoxin belong to the same group of toxin-antitoxin system.

When the bacterial host cell in (I) exhibits a suicide effect and the bacterial host cell in (II) grows normally and the plasmid is stably passaged, an effective toxin-antitoxin system is obtained by screening.

The present application is further illustrated below by using examples, but the present application is not limited to the scope of the described examples. The experimental methods in the following examples without specified specific conditions should be selected according to conventional methods and conditions, or according to instructions of products.

### Example 1 Inducing insertion of lethal toxin gene expression cassette into bacterial genome for preparation of plasmid production strains

### (1) Selection of insertion position in GT115 genome

An internal of an *Escherichia coli lacZ* gene is a common position where an exogenous expression gene is inserted into a genome. According to a genome sequence of a model strain MG1655 in a public database, primers were designed, DNA of a *lacZ* gene in an *Escherichia coli* GT115 genome (purchased from InvivoGen) was amplified and sequenced, and sequence information of the insertion position was confirmed. Through sequencing verification, the *lacZ* region (sequence shown below) of the GT115 strain was identical to that of MG1655. The region (underline represented a sequence replaced by a toxin) was chosen as the position for toxin insertion:

TCATACAGAACTGGCGATCGTTCGGCGTATCGCCAAAATCACCGCCGTAA
GCCGACCACGGGTTGCCGTTTTCATCATATTTAATCAGCGACTGATCCACCCAGTCCC
AGACGAAGCCGCCCTGTAAACGGGGATACTGACGAAACGCCTGCCAGTATTTAGCGA
AACCGCCAAGACTGTTACCCATCGCGTGGGCGTATTCGCAAAGGATCAGCGGGCGCG
TCTCTCCAGGTAGCGAAAGCCATTTTTTGATGGACCATTTCGGCACAGCCGGGAAGG
GCTGGTCTTCATCCACGCGCGCGTACATCGGGCAAATAATATCGGTGGCCGTGGTGTC

GGCTCCGCCGCCTTCATACTGCACCGGGCGGGAAGGATCGACAGATTTGATCCAGCG
ATACAGCGCGTCGTGATTAGCGCCGTGGCCTGATTCATTCCCCAGCGACCAGATGATC
ACACTCGGGTGATTACGATCGCGCTGCACCATTCGCGTTACGCGTTCGCTCATCGCCG
GTAGCCAGCGCGGATCATCGGTCAGACGATTCATTGGCACCATGCCGTGGGTTTCAAT
ATTGGCTTCATCCACCACATACAGGCCGTAGCGGTCGCACAGCGTGTACCACAGCGG
ATGGTTCGGATAATGCGAACAGCGCACGGCGTTAAAGTTGTTCTGCTTCATCAGCAGG
ATATCCTGCACCATCGTCTGCTCATCCATGACCTGACCATGCAGAGGATGATGCTCGT
GACGGTTAACGCCTCGAATCAGCAACGGCTTGCCGTTCAGCAGCAGACCATTTT
CAATCCGCACCTCGCGGAAACCGACATCGCAGGCTTCTGCTTCAATCAGCGTGCCGT
CGGCGGTGTGCAGTTCAACCACCGCACGATAGAGATTCGGGATTTCGGCGCTCCACA
GTTTCGGGTTTTCGACGTTCAGACGTAGTGTGACGCGATCGGCATAACCACCACGCTC
ATCGATAATTTCACCGCCGAAAGGCGCGGTGCCGCTGGCGACCTGCGTTTCACCCTG
CCATAAAGAAACTGTTACCCGTAGGTAGTCACGCAACTCGCCGCACATCTGAACTTC
AGCCTCCAGTACAGCGCGGCTGAAATCATCATTAAAGCGAGTGGCAACATGGAAATC
GCTGATTTGTGTAGTCGGTTTATGCAGCAACGAGACGTCACGGAAAATGCCGCTCATC
CGCCACATATCCTGATCTTCCAGATAACTGCCGTCACTCCAGCGCAGCACCATCACCG
CGAGGCGGTTTTCTCCGGCGCGTAAAAATGCGCTCAGGTCAAATTCAGAC (SEQ ID
NO: 47).

FIG. 2 shows a schematic diagram of inserting a toxin gene into the GT115 genome.

### (2) Cloning of toxin gene under control of arabinose operon

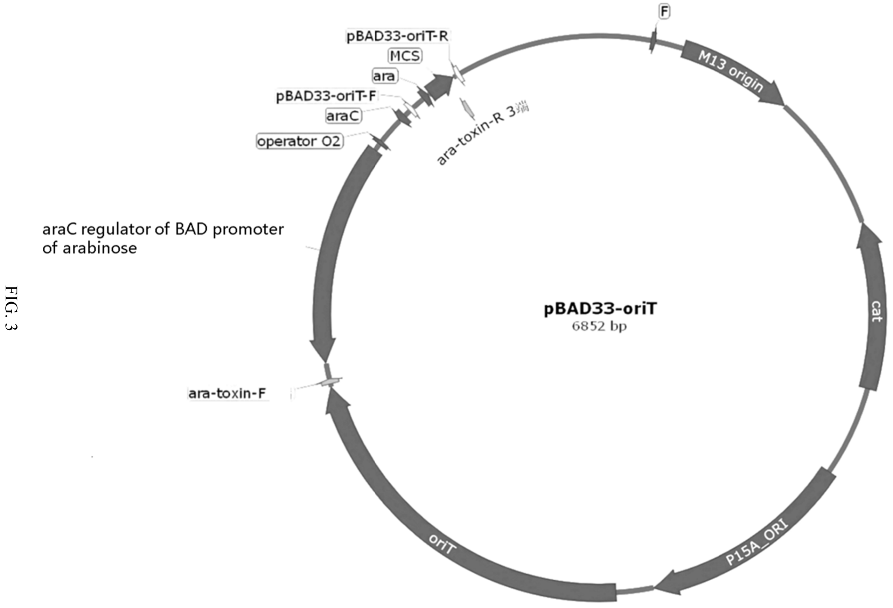

A toxin gene DNA in a TA System was subjected to gene synthesis. The DNA fragment was cloned into a pBAD33-oriT plasmid (between Xba I and Hind III restriction enzyme cutting sites, ORF direction from Xba I to Hind III) inducibly expressed by arabinose to obtain a toxin induction expression element in the form of a plasmid shown in FIG. 3.

Then, positive transformants were verified by PCR. The results are shown in FIG. 4, where the sample sequence is: 1444, ccdB (43009), 1102, hepT, vagD1, 0636, and an EcccdB empty control. Through sequencing identification, sequences of 7 pBAD33-oriT induction expression plasmids with toxins were all correct.

### (3) Fusion of toxin gene and gentamicin (Gm) resistance selectable marker

The pBAD33-oriT plasmid with the toxin gene was used as a template, a complete arabinose operon (1428 bp+toxin gene) was amplified by using ara-toxin-F and ara-toxin-R primers, and then the complete arabinose operon was linked with a gentamicin (Gm) resistance gene sequence with FRT sequences on two sides through fusion PCR to obtain a DNA fragment for inserting the GT115 genome. The positions of elements in the fragment and the fusion PCR primers were shown in FIG. 5 (where each toxin gene was already in the MCS), where up-ram and down-arm parts at two ends indicated upper and lower arm sequences recombined with the GT115 genome.

### (4) Insertion of fusion toxin gene fragment

### 4.1 Preparation of GT115 electroporation competent cells

A pKD46 plasmid was transferred into a GT115 host by a chemical transformation method, positive transformants were screened by using Car300 (carbenicillin, final concentration of 300 µg/mL) at 30°C, the positive transformant GT115/pKD46 monoclonal bacteria were inoculated into a liquid LB medium for overnight culture at 30°C, transferred to a triangular flask containing 25 mL of fresh LB+Car300 liquid medium at a ratio of 1:100 the next day, and cultured in a shaker at 30°C until OD was about 1.0. after arabinose with the final concentration of 3 mg/mL was added for continuous induced culture for 2 h, the bacteria were immediately cooled at 4°C, collected at 4°C under centrifugation of 5000 rpm/min, washed twice by precooled 10% glycerol, and finally dissolved in 10% glycerol and placed in a refrigerator at -80°C for standby use.

### 4.2 Electroporation and screening of toxin-inserted strains

About 1 µg of a fusion toxin gene fragment and 100 µl of GT 115/pKD46 competent cells were mixed in an electroporation cuvette and subjected to an electric shock for 5 ms at 1,250 V by using an electroporator. Then 900 µl of the liquid LB medium was immediately mixed with the product to be sucked out and the mixture was placed in an incubator at 30°C for standing for 8 h, such that the toxin gene fragment and the GT115 genome were subjected to homologous recombination.

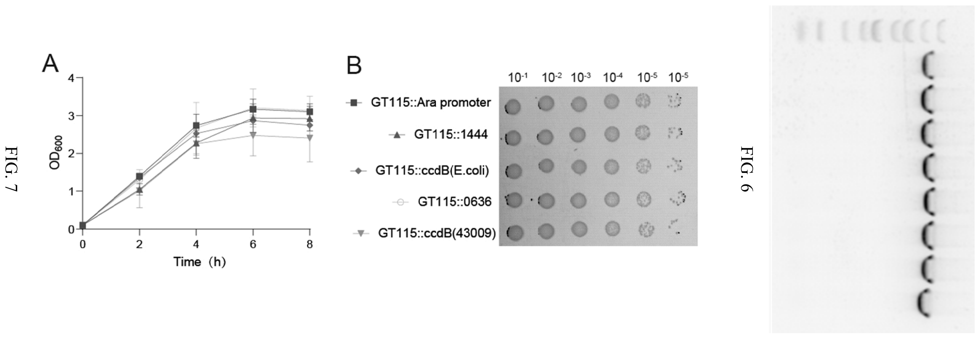

The mixture was centrifuged to remove 900 µl of a supernatant, the remaining 100 µl of supernatant was used to resuspend the bacteria, the bacteria were coated on a solid LB+Gm30 (final concentration of gentamicin of 30 µg/ml)+Glu20 (final concentration of glucose of 2 mg/ml) plate, glucose was added to avoid toxin leakage caused by background expression of the arabinose operon as much as possible, and the plate was placed at 37°C for overnight culture, and under the culture condition, a temperature-sensitive pKD46 plasmid was automatically lost. Single colonies were picked and cultured in liquid LB+Gm30 (final concentration of gentamicin of 30 µg/ml)+Glu20 (final concentration of glucose of 2 mg/ml) at 37°C. The PCR identification was performed using verification primers of the GT115 genome shown in FIG. 6. Sample sequence: 1444, ccdB (43009), 1102, hepT, vagD1, 0636, EcccdB, and an arabinose operon control.

The size of a GT115 genome control band was 1,018 bp (not shown). The size was 2,850 bp after the arabinose operon not containing the toxin gene was inserted. The size of the arabinose operon with the toxin gene inserted was 2,850+toxin orf.

The sequence of the inserted toxin in the GT115 genome was all correct through sequencing identification.

### 4.3 Identification of pKD46 plasmid loss in GT115::toxin strains

The GT115 strain correctly inserted with the toxin gene was streaked on a solid LB+Gm30 (final concentration of gentamicin of 30 µg/ml)+Glu20 (final concentration of glucose of 2 mg/ml) plate and cultured at 37°C to obtain single colonies. Then the single colonies were picked and simultaneously patched correspondingly on a Gm30+Glu20 plate and a Car300+Glu20 plate. The single colonies growing normally on the Gm30+Glu20, but not growing on the Car300+Glu20, were obtained, namely, the GT115 toxin-inserted strain losing the pKD46 plasmid.

### Example 2 Screening of toxin genes of TA systems capable of efficiently inducing expression of lethal production strains

Toxin lethal genes in 6 pairs of potential TA systems were selected and subjected to genomic integration **(Table 1),** and were respectively a TA pairing system of RelB/RelE derived from *Roseivirga spongicola_*GF047 (shown in SEQ ID NOs: 5-6), a TA pairing system of CcdB/CcdA (Vibrio) derived from *Vibrio* sp. SCSIO 43009 (shown in SEQ ID NOs: 14-15), a TA pairing system of 1444 and 1445 of SO_1444/SO_1445 derived from*Shewanella oneidensis* MR-1 (shown in SEQ ID NOs: 23-24), a TA pairing system of VagC1/VagD1 derived from *E.coli* 14EC022-1 (shown in SEQ ID NOs: 25-26), a TA pairing system of PrpT/PrpA derived from *Pseudoalteromonas* sp. SCSIO 6842 (shown in SEQ ID NOs: 29-30), a TA pairing system of HepT/MntA derived from E.coli 14EC01 (shown in SEQ ID NOs: 29-30), and a TApairing system of CcdA/CcdB(*E.coli*) derived from *E.coli* 0157 (shown in SEQ ID NOs: 35-36).

Firstly, the toxin genes were respectively fused with an arabinose-induced promoter and inserted into the *lacZ* gene of the GT115 genome by using a homologous recombination method (specifically described in Example 1) to obtain 7 GT115::toxin strains: GT115::GF_0636, GT115::*SO_1444,* GT115*::ccdB*₄₃₀₀₉*, GT115::prpT,* GT115:*:vagD1,* GT115*::ccdB*_{E.coli} and *GT115::hepT.*

**Table 1 Screening effects of 6 candidate toxin-antitoxin systems**

| No. | Candidate TA system | Toxin gene | Source strain | Size of antitoxin | Whether to induce a lethal production strain | Size of prokaryotic backbone |
|---|---|---|---|---|---|---|
| 1 | GF_0636/GF_0 637 | GF_0636 | GF047 | 207 bp | Yes | 540 bp |
| 2 | VagC₁/VagD₁ | VagD₁ | *E.coli* 14EC022-1 | 231 bp | No | 564 bp |
| 3 | SO_1444/SO_1 445 | SO_1444 | *Shewanella oneidensis* MR-1 | 285 bp | Yes | 618 bp |
| 4 | PrpT/PrpA | PrpT | *Pseudoalteromona* s sp. SCSIO 6842 | 261 bp | No | 594 bp |
| 5 | HepT/MntA | HepT | *E.coli* 14EC01 | 291 bp | No | 624 bp |
| 6 | CcdA/CcdB (43009) | CcdB (43009) | *Vibrio alginolyticus* SCSIO 43009 | 246 bp | Yes | 579 bp |
| Contr ol | CcdA/CcdB (*E.coli*) | CcdB (*E.coli*) | *E.coli* O157 | 219 bp | Yes | 552 bp |

The toxin genes of the TA systems suitable for antibiotic resistance gene-free plasmid production strains were screened by detecting the lethality of the strain under the toxin induction expression condition. The glucose can inhibit a pBAD promoter and prevent toxin leakage expression. The arabinose can activate the pBAD promoter and induce a large amount of toxin expression. Therefore, 1% of the overnight-cultured GT115::toxin strains were respectively transferred to an LB medium containing 0.2% glucose and 0.3% arabinose, and patched correspondingly on an LB plate after 2h of the induction, and the survival rate of the bacteria was calculated.

The growth of the overnight-cultured strains is shown in FIG. 7 and the survival rate of the bacteria after the induction is shown in FIG. 8.

The results showed that except for the toxin of CcdB (*E.coli*) of the control group, under no induction of the toxin of only three TA systems of SO_1444, CcdB (43009) and GF_0636, the bacteria had no obvious growth defect (FIG. 7). However, after the induction, the strains all exhibited an obvious suicide effect and had the lethality exceeding 99%, which meets the requirement for inducing a lethal production strain of the antibiotic resistance gene-free plasmid and may be used for a subsequent experiment to verify the production stability of the matched plasmid in the screened strains. However, the cytotoxicity was not obvious after the toxin genes in the three pairs of TA systems of VagC₁/VagD₁, PrpT/PrpA and HepT/MntA were expressed. The production strains hardly died, such that the three pairs of TA systems were obviously not suitable for the production of the antibiotic resistance gene-free plasmid.

### Example 3 Preparation of clones of antibiotic resistance gene-free miniplasmid mother vector, and effect on growth stability of production strains

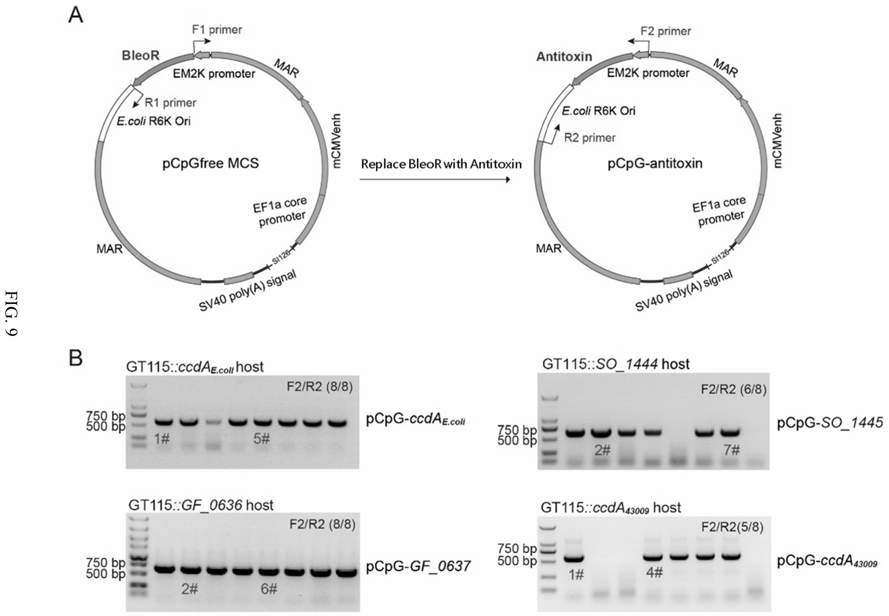

The results of Example 2 demonstrated that efficient suicide of plasmid-free production strains can only be efficiently induced by induced expression of suitable toxin types. The present example demonstrated that only when the matched plasmid expressing the antitoxin gene was present in the cells, the production strains expressing the antitoxin can survive. Based on this, when GT115::toxins were cultured, the arabinose was added to the medium to induce toxin expression, then the corresponding antitoxin gene was inserted into the plasmid, and only the strain with the correct insertion and expression of the antitoxin gene survived. A basic backbone of a plasmid pCpGfree MCS widely used for eukaryotic expression was used. A primary plasmid bleomycin resistance gene *(bleoR)* was replaced with the antitoxin gene matched by the toxin production strain (A in FIG. 9). Specific operations were as follows:

PCR amplification was performed to obtain a linearized pCpGfree MCS plasmid;
a CpG DNA motif-free antitoxin gene expression cassette with a 5' end carrying a 20-bp sequence homologous with a vector terminal was subjected to gene synthesis;
the antitoxin gene was recombined to the pCpGfree MCS plasmid by using a commercial recombinant kit to replace a bleomycin resistance expression cassette;
a recombinant product CaCl₂ was transformed into GT115::toxin competent cells and the competent cells were coated on a plate of LB+Gm10+0.6% Ara (arabinose);
whether the target gene was inserted in transformants was verified by using primer PCR; the positive transformants were further purified;

F2/R2 amplified a fragment comprising an R6K replicon of the antibiotic resistance gene-free miniplasmid and the antitoxin gene (shown in A in FIG. 5); and 8 clones were picked randomly from the LB+Gm10+0.6% Ara plate and verified by PCR using the F2/R2 primers. The results showed that the antitoxin-inserted antibiotic resistance gene-free plasmids pCpG*-ccdA_{E.coli},* pCpG-*SO_1445,* pCpG*-ccdA₄₃₀₀₉,* and pCpG-*GF_0636* can be correspondingly obtained from GT115*::ccdB_{E.coli},* GT115*::SO_1444,* GT115::*ccdB*₄₃₀₀₉, and *GT115::GF_0636* toxin expression production strains, with the frequencies of (8/8) 100%, (6/8) 75%, (8/8) 100%, and (5/8) 62.5% respectively (B in FIG. 9).

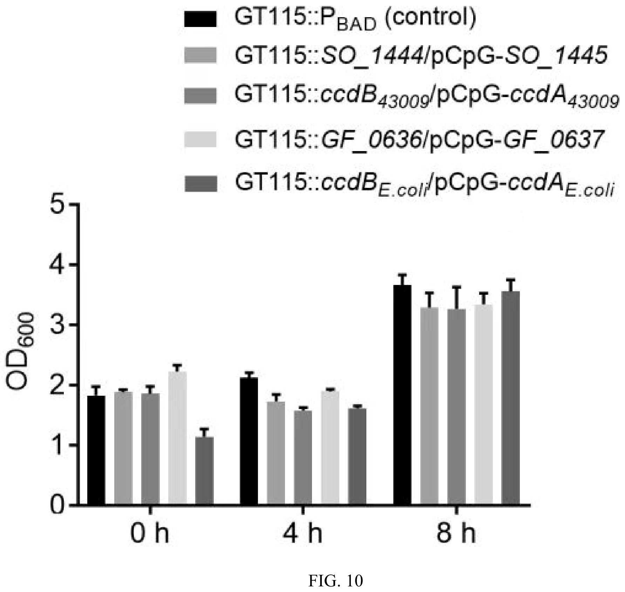

To detect whether GT115::toxin/pCpG-antitoxin had an effect on the growth of the strains, GT115 (GT115::pBAD) integrated with the pBAD promoter was used as a control, and the OD₆₀₀ values of the strains *GT115::SO_1444*/*pCpG-SO_1445,* GT115:*:ccdB*₄₃₀₀₉/pCpG*-ccdA₄₃₀₀₉, GT115::GF_0636*/*pCpG-GF_0637,* and GT115:*:ccdB_{E.coli}*/pCpG-*ccdA_{E.coli}* in the LB medium containing 0.3% arabinose cultured for 0 h, 4 h and 8 h were respectively determined. The results indicated that the strains containing the four pairs of TA systems had no significant growth difference compared to the control (FIG. 10).

**Example 4 Method and efficiency of cloning exogenous gene from pCpG-antitoxin antibiotic resistance gene-free miniplasmids**

To verify the efficiency of cloning an exogenous gene from the pCpG-antitoxin plasmids, an *egfp* gene was distributionally inserted into pCpG*-ccdA_{E.coli}, CpG-SO_1445,* pCpG-*ccdA₄₃₀₀₉,* and pCpG*-GF_0637.* Specific operations were as follows:
1. a pCpG-antitoxin was used as a template and a linearized pCpG-antitoxin plasmid was reversely amplified by pCpG-BglII-F/pCpG-NheI-R primers;
2. an *egfp* gene was amplified by EGFP-F/-R primers and homologous fragments at ends of pCpG-antitoxin insertion sites were introduced into two ends of the EGFP-F/-R primers;
3. the *egfp* was inserted between BglII and NheI on the pCpG-antitoxin by using a commercial recombinase;
4. a recombinant product was transformed into GT115::toxin competent cells and the competent cells were coated on a plate of LB+Gm10+0.6% Ara (arabinose); and
5. whether the antitoxin gene and the *egfp* were inserted into transformants was verified by PCR using two pairs of F2/R2 and EGFP-F/-R primers; the positive transformants were further purified;

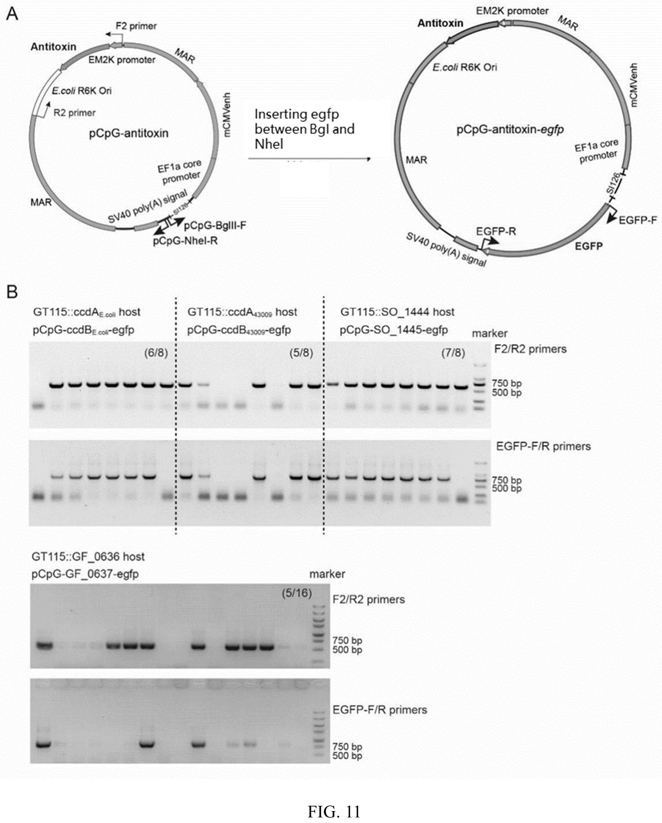

F2/R2 amplified a fragment comprising the R6K replicon and the antitoxin gene, wherein codons of the antitoxin gene expression cassette were optimized to be free of a CpG DNA motif; EGFP-F/-R amplified whether the *egfp* was inserted (shown in FIG. 11A); and 8 or 16 clones were picked randomly from the LB+Gm10+0.6% Ara plate and verified by PCR using the F2/R2 and EGFP-F/R primers. The results showed that in the GT115*::ccdB_{E.coli},* GT*115::SO_1444,* GT115::*ccdB*₄₃₀₀₉ and *GT115::GF_0636* toxin expression strains, the *egfp* may be successfully inserted into the corresponding antibiotic resistance gene-free plasmids pCpG*-ccdA_{E.coli},* pCpG-*SO_1445,* pCpG-*ccdA₄₃₀₀₉,* and pCpG-GF_*0637* to obtain recombinant plasmids pCpG*-ccdA_{E.coli}*-*egfp,* pCpG*-SO_1445-egfp,* pCpG*-ccdA₄₃₀₀₉-egfp,* and pCpG*-GF_0637-egfp,* with the efficiencies of (6/8) 75%, (5/8) 62.5%, (7/8) 87.5%, and (5/16) 31.25%, respectively (B in FIG. 11).

### Example 5 Detection of production and passage stability of antibiotic resistance gene-free miniplasmids pCpG-antitoxin

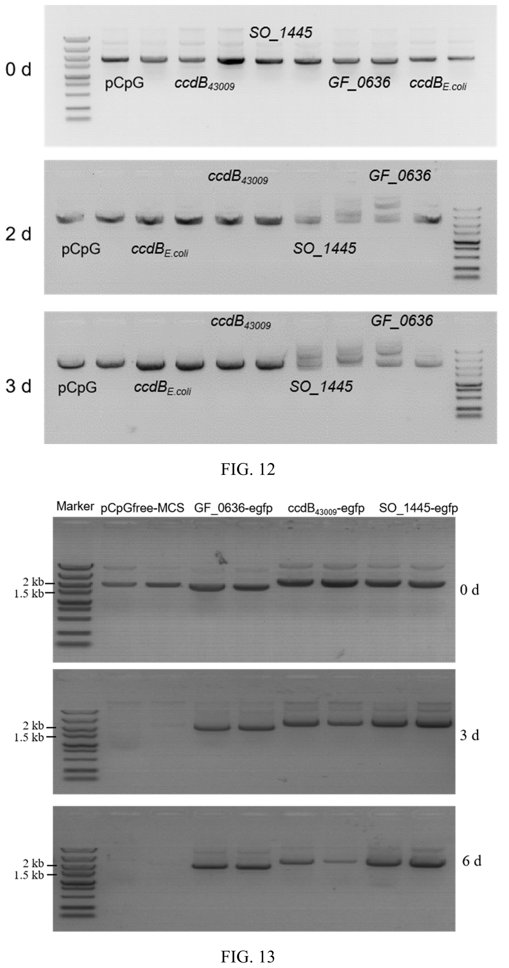

In order to detect the production and passage stability of the antibiotic resistance gene-free miniplasmids in the production strains of the present application, the pCpGfree-MCS plasmid was used as a control, and *pCpG-SO_1445,* pCpG*-ccdA_{E.coli},* pCpG*-ccdA₄₃₀₀₉* and pCpG-GF_0637 plasmids not inserted with antitoxin expression cassette DNA were subjected to subculture and stability detection. 2 single clones were randomly selected from each sample and inoculated into a liquid LB for overnight culture, 1/100 of the bacteria were transferred to a triangular flask of 25 mL of the liquid LB medium for antibiotic-free subculture, the bacteria were transferred once every 12 h and collected, the plasmids were extracted, the concentration of plasmid DNA was detected by agarose gel, the plasmid concentration was determined. DNA gel graphs and the plasmid concentration distribution are shown in **FIG. 12** and **Table 2.** The results showed that in a medium culture system of 25 mL of the LB medium, the subculture of the antibiotic resistance gene-free miniplasmids pCpG-antitoxin was relatively stable, particularly the pCpG*-ccdA₄₃₀₀₉* plasmid. After 2 days of the subculture, the plasmid yield was higher than that of the control pCpGfree-MCS and the pCpG*-ccdA_{E.coli}.*

**Table 2 Determination of plasmid concentration after continuous transfer and culture of antibiotic resistance gene-free miniplasmids for 3 days (unit: ng/µl).**

| Sample | pCpG | *ccdA₄₃₀₀₉* | *SO_1445* | GF_0637 | *ccdA_{E. coli}* |
|---|---|---|---|---|---|
| replicate1 | 59.64 | 103.24 | 48.68 | 49.88 | 73.32 |
| replicate2 | 71.56 | 68.68 | 50.76 | 76.04 | 79.96 |
| Average value | 65.6 | 85.96 | 49.72 | 62.96 | 76.64 |

**Example 6 Detection of production and passage stability of plasmids pCpG-antitoxin-egfp**

To detect the stability of the antibiotic resistance gene-free miniplasmids after inserting an eGFP exogenous gene, the pCpGfree-MCS plasmid was used as a control (toxin-free host was used) and pCpG*-SO_1445-egfp,* pCpG-*ccdA₄₃₀₀₉-egfp,* and pCpG*-GF_0637-egfp* were subcultured. Firstly, the four plasmids were purified and the bacteria were preserved. 2 single clones were randomly selected and subjected to antibiotic-free subculture by using an LB medium, the bacteria were transferred once every 12 h and collected, the plasmids were extracted, the concentration of plasmid DNA was detected by agarose gel, and the plasmid concentration was determined. After a total passage of 6 days in the experiment, the results showed that the three single strains of the pCpG*-SO_1445-egfp,* the pCpG*-ccdA₄₃₀₀₉-egfp* and the pCpG*-GF_0637-egfp* were always stable and the yield was higher than that of the control pCpGfree-MCS (FIGS. 13 and 14, and Table 3).

**Table 3 Original data of plasmid concentration corresponding to FIG. 10 of egfp-inserted antibiotic resistance gene-free miniplasmids after continuous subculture for 6 days**

| | | pCpG | *ccdA₄₃₀₀₉-egfp* | *GF_0637-egfp* | *SO_1445-egfp* |
|---|---|---|---|---|---|
| 0 day | replicatel | 45.75 | 124.05 | 78.35 | 78.95 |
| | replicate2 | 54.25 | 118.65 | 82.55 | 99.65 |
| | Average value | 50 | 121.35 | 80.45 | 89.3 |
| 3 days | replicate1 | 7.65 | 77.65 | 80.65 | 76.15 |
| | replicate2 | 8.25 | 49.95 | 76.85 | 82.45 |
| | Average value | 7.95 | 63.8 | 78.75 | 79.3 |
| 6 days | replicate1 | 8.5 | 81.9 | 71.9 | 75 |
| | replicate2 | 12.1 | 25.4 | 74.4 | 94.4 |
| | Average value | 10.3 | 53.65 | 73.15 | 84.7 |

pCpG:pCpGfree-MCS, *ccdA₄₃₀₀₉-egfp:* pCpG*-ccdA₄₃₀₀₉-egfp, GF_0637-egfp:*pCpG-*GF_0637-egfp,* and *SO_1445-egfp: pCpG-SO_1445-egfp.*

### Example 7 Effectiveness of truncated antitoxins in production of antibiotic resistance gene-free miniplasmids

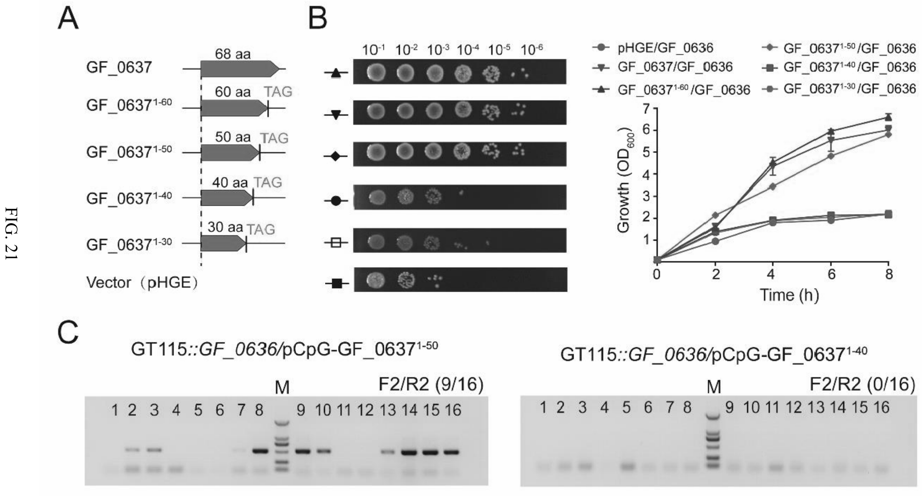

To further reduce the DNA lengths of the antibiotic resistance gene-free miniplasmids, an expression cassette of an antitoxin GF_0637 was subjected to varying degrees of C-terminal truncation, including 60 aa (SEQ ID NO: 2), 50 aa (SEQ ID NO: 1), 40 aa (SEQ ID NO: 43) and 30 aa (SEQ ID NO: 44) (A in FIG. 21). Plasmids carrying the non-truncated and truncated antitoxin GF_0637 were co-transferred to *Escherichia coli* with a plasmid carrying a toxin GF_0636. Overexpression of the GF_0636 and an empty vector in the *Escherichia coli* resulted in severe growth inhibition, whereas co-expression of the GF_0637 completely neutralized the GF_0636 (B in FIG. 21). As expected, the results showed that different C-terminal truncations of the GF_0637 exhibited different toxicity antagonistic abilities of the GF_0636. Growth and CFU results indicated that the GF_0637 contained truncated fragments of 60 amino acids or 50 amino acids as a full-length antitoxin. However, the GF_0637 was truncated and shortened to 40 amino acids, and when shortened to 30 amino acids, completely lost the ability to neutralize toxin toxicity (B in FIG. 21). To confirm the results, based on a GF_0636/GF_0637 selection system, GF_0637 truncated GF_06371-50 and GF_06371-40 were cloned and inserted into a pCpGfree plasmid to replace the full-length GF_0637. The efficiency was tested. As expected, the first 50 amino acids of the GF_0637 were the minimum to exert an antitoxin function in DNA clones (C in FIG. 21).

Based on similar strategies and principles, a ccdA 43009 expression cassette was also correspondingly truncated. The results showed that the truncation of the 2nd-9th amino acids and/or the 78th-81st amino acids of the ccdA 43009 was also effective. The truncated sequences are shown in SEQ ID NOs: 9-11. Therefore, it is well known to those skilled in the art that the truncation of the antitoxins to some extent may still suitably preserve the original function to maintain the production of the antibiotic resistance gene-free miniplasmids.

### Example 8 Use and effect of antibiotic resistance gene-free miniplasmids as exogenous gene expression vectors in eukaryotic cells

### Research purpose:

In CHO cells, the biological functions of the antibiotic resistance gene-free miniplasmids of the pCpG-ccdA43009-egfp and the pCpG-GF_0637-egfp, and the pCpGfree-MCS backbone plasmid with an antibiotic resistance gene were compared. The expression quantity and the expression durability of the different plasmids were observed.

### Research method:

A Jurkat cell line was used, belonged to immune cells and electroporated with about 4 µg of the plasmids. The cells were continuously observed for one week and subjected to fluorescence photography. The expression durability was subjected to flow cytometry for 2, 4 and 6 days. Cell grouping is shown in the table below:

| Grouping Jurkat cells | Name of plasmid (for example 4 µg) | Dose |
|---|---|---|
| 1 Antibiotic resistance gene-free miniplasmid | pCpG-ccdA43009-egfp | 4 µg |
| 2 Antibiotic resistance gene-free miniplasmid | pCpG-GF_0637-egfp | 4 µg |
| 3 pCpGfree-MCS backbone with antibiotic resistance gene | pCpGfree-dCGEGFP | 4 µg |

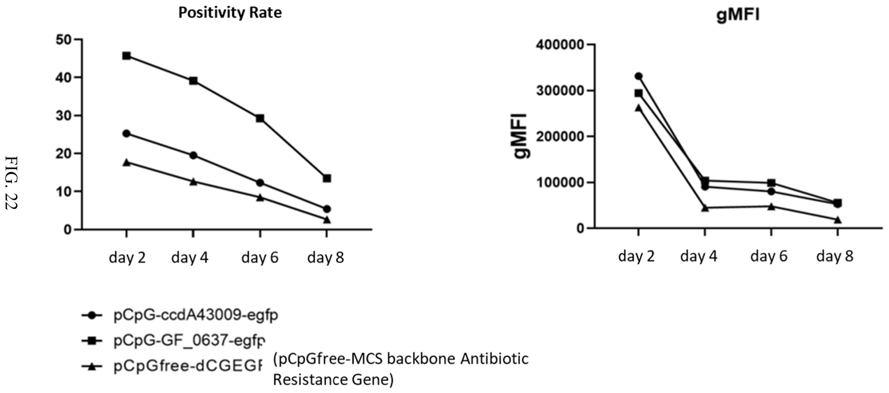

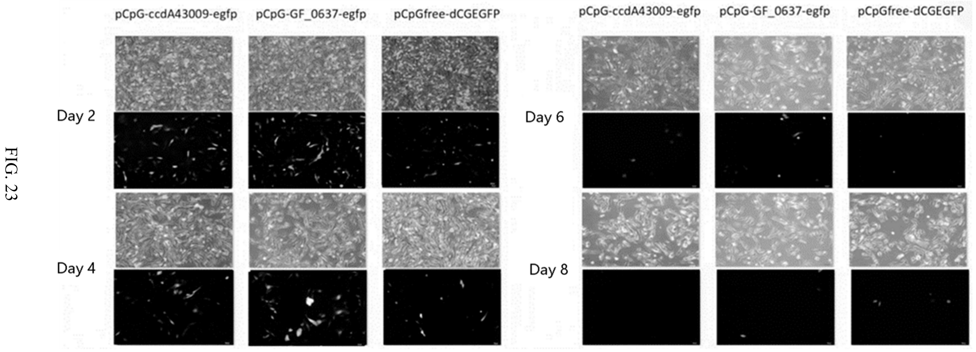

The test results are shown in FIGS. 22-23. The results of the fluorescence photography and flow cytometry showed that the expression level and durability of the antibiotic resistance gene-free miniplasmid grouped cells of the present application were better, indicating that the antibiotic resistance gene-free miniplasmids of the present application contribute to the expression of the plasmids in eukaryotic cells.

### Example 9 Use and effect of antibiotic resistance gene-free miniplasmids as gene integration donors in eukaryotic cells

In the example, an antibiotic resistance gene-free miniplasmid-loaded eGFP expression cassette was integrated into Jurkat and PBMCs by using a ZB transposase to verify the effect of gene integration transposition efficiency mediated by an antibiotic resistance gene-free miniplasmid transposon.

The ZB transposase was a transposase in CN105018523B. ZB transposon vectors corresponding to the ZB transposase were respectively the antibiotic resistance gene-free miniplasmid and a puc57 plasmid, wherein the antibiotic resistance gene-free miniplasmid pTini-ZB-dCGEGFP was a pCpGfree MCS-0637 plasmid loaded with an eGFP expression cassette and an R6K replicon was used. A control plasmid was the puc57 replicon plasmid pZB-dCG EGFP V3.0 being the puc57 plasmid loaded with an eGFP expression cassette. The ZB transposase was in the form of mRNA in the amount of 20 µg. The amounts of the ZB transposon vectors pTini-ZB-dCGEGFP and pZB-dCG EGFP V3.0 were both 4 µg. The experiment was divided into 4 groups, namely pZB-dCG EGFP V3.0, pZB-dCG EGFP V3.0+ZB mRNA, pTini-ZB-dCGEGFP, and pTini-ZB-dCGEGFP+ZB mRNA, with 3 experimental batches per group.

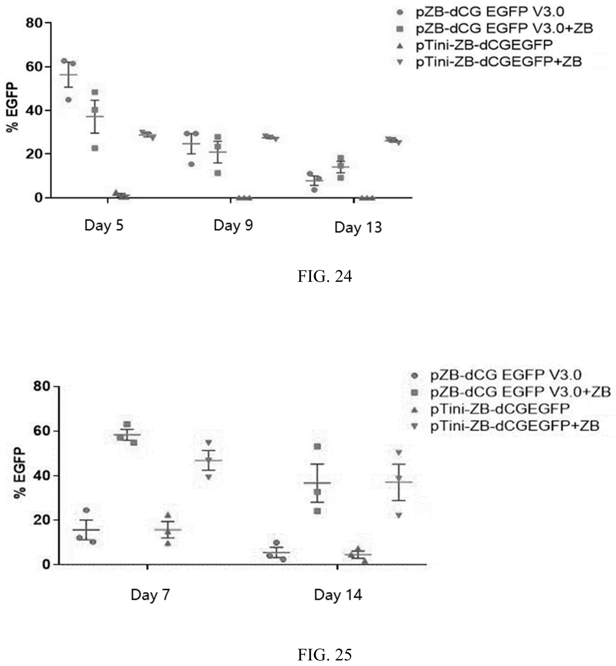

The ZB transposase and transposon vectors were electroporated into the Jurkat cells and PBMCs by using a Lonza2b electroporation program, and cultured and passaged continuously by using a complete medium. Sampling was respectively performed on days 5, 9 and 13 after the electroporation of the Jurkat cells, and the EGFP expression was detected by flow cytometry. Sampling was respectively performed on days 7 and 14 after the electroporation of the PBMCs, and the EGFP expression was detected by flow cytometry. The results are shown in FIGS. 24 and 25. It can be seen from the figure that on day 13, the antibiotic resistance gene-free miniplasmid had a superior final positive rate of the Jurkat cells; and on day 14, the antibiotic resistance gene-free miniplasmid had a superior final positive rate of the PBMCs. Therefore, it can be known from the results of the example that the antibiotic resistance gene-free miniplasmid of the present application can improve the gene integration transposition efficiency mediated by the transposon by 100%.

### Example 10 TA System for production of transcription template plasmid and TA System for preparation of antibiotic resistance gene-free puc57 replicon plasmid with reduced size

An ampicillin resistance gene expression cassette of an original mRNA transcription template plasmid pT7-6.5-MDR1-EGFP (shown in SEQ ID NO: 45) was replaced by an antitoxin expression cassette to obtain a plasmid pucTA-6.5A-MDR1-EGFP-90A (shown in SEQ ID NO: 46).

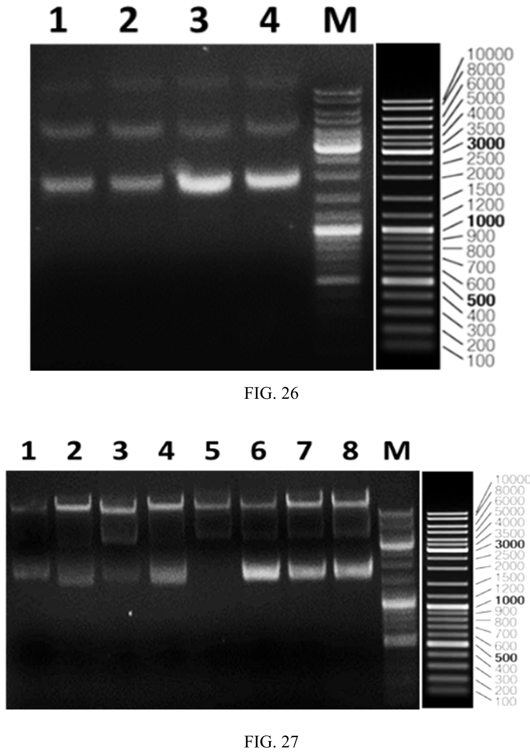

A pucTA-6.5A-MDR1-EGFP-90A strain was streaked on a TB solid plate containing 0.3% L-arabinose, single clones were picked and respectively inoculated into 2 ml of a TB medium containing 0.3% L-arabinose for overnight culture, then 1.4 ml of a culture solution was respectively taken, and a small amount of the plasmid was extracted and quantified by a nanodrop ultramicro spectrophotometer. The yields of the plasmids of clones No. 1 to No. 4 were 3.7 µg/ml, 3.8 µg/ml, 4.8 µg/ml, and 4.7 µg/ml respectively. The plasmids were detected through agarose gel electrophoresis. The plasmids extracted from the 4 clones met the expected size and had relatively high purity as shown in FIG. 26.

In addition, one clone was selected for passage, each passage was a 24-hour culture period, 9 passages were continuously cultured, and a small amount of the plasmids were extracted. The plasmid yield was between 3 µg/ml and 5 µg/ml. The plasmids were detected by the agarose gel electrophoresis shown in FIG. 27. The results indicated that under the screening pressure of 0.3% L-arabinose, the plasmid may be stably present in the bacteria.

The experimental results showed that the antibiotic resistance gene-free production system may also be successfully used in plasmids of similar types of puc57, ColE1 and pMB1. The replacement of the antibiotic expression cassette can reduce the size of the plasmid and can further improve the safety of plasmid production.

## Claims

1. An antibiotic resistance gene-free miniplasmid, comprising a nucleotide sequence encoding an antitoxin protein, and a replicon, wherein
an amino acid sequence of the antitoxin protein comprises the following sequence: (1) an amino acid sequence shown in SEQ ID NO: 1, or an amino acid sequence having one or more mutations of E24D, I36V and V43I compared with SEQ ID NO: 1; or (2) an amino acid sequence shown in SEQ ID NO: 9, or an amino acid sequence having one or more mutations of T6I, T43A, K47E, A50S, E51D, G52A and N54K compared with SEQ ID NO: 9; and
a length of the replicon is ≤ 800 bp, preferably ≤ 600 bp or ≤ 300 bp.

2. The antibiotic resistance gene-free miniplasmid according to claim 1, wherein the amino acid sequence of the antitoxin protein is shown in SEQ ID NO: 1, SEQ ID NO: 2, SEQ ID NO: 3, SEQ ID NO: 9, SEQ ID NO: 10, SEQ ID NO: 11 or SEQ ID NO: 12.

3. The antibiotic resistance gene-free miniplasmid according to claim 1, wherein the replicon is selected from ColE1, ColE2, pMB1, pSC101, RSF, R6K, pUC57, RK2, and p15A, preferably R6K or pUC57.

4. The antibiotic resistance gene-free miniplasmid according to any one of claims 1-3, wherein a length of a plasmid backbone of the antibiotic resistance gene-free miniplasmid is ≤ 1,000 bp, preferably ≤ 900 bp, ≤ 800 bp or ≤ 600 bp.

5. The antibiotic resistance gene-free miniplasmid according to any one of claims 1-4, wherein the nucleotide sequence encoding an antitoxin protein does not comprise a CpG motif and preferably, the nucleotide sequence encoding an antitoxin protein is shown in SEQ ID NO: 7 or SEQ ID NO: 16.

6. The antibiotic resistance gene-free miniplasmid according to any one of claims 1-5, wherein a nucleotide sequence of the replicon does not comprise a CpG motif.

7. The antibiotic resistance gene-free miniplasmid according to any one of claims 1-6, wherein the antibiotic resistance gene-free miniplasmid comprises a structured DNA sequence selected from a polyA repeat, an SV40 origin of replication, a viral LTR, a lentiviral LTR, a retroviral LTR, a transposon IR/DR, an AAV ITR, a transposon ITR, a CMV enhancer, and an SV40 enhancer.

8. The antibiotic resistance gene-free miniplasmid according to claim 7, wherein the antibiotic resistance gene-free miniplasmid is selected from a Sleeping Beauty transposon vector, a PiggyBac transposon vector, a ZB transposon vector, a PS transposon vector, a Tol2 transposon vector, and a polyA-containing mRNA vector.

9. The antibiotic resistance gene-free miniplasmid according to any one of claims 1-8, wherein the antibiotic resistance gene-free miniplasmid comprises an exogenous gene, and preferably, the exogenous gene encodes a chimeric antigen receptor and/or encodes an antibody.

10. A recombinant host cell, comprising the antibiotic resistance gene-free miniplasmid according to any one of claims 1-9.

11. A method for preparing a cell, comprising introducing the antibiotic resistance gene-free miniplasmid according to any one of claims 1-9 into a cell.

12. The method according to claim 11, wherein the cell is from an animal, for example, a vertebrate or an invertebrate, preferably a mammal, further preferably a human; and/or the cell is an immune cell, preferably a T cell.

13. The method according to claim 11 or 12, wherein the introducing comprises transfecting the cell by electroporation, microinjection, calcium phosphate precipitation, a cationic polymer, a dendrimer, a liposome, microparticle bombardment, fugene, direct acoustic wave loading, cell extrusion, optical transfection, protoplast fusion, impalefection, magnetic transfection, nucleofaction, or any combination thereof, preferably by electroporation.

14. A pharmaceutical composition, comprising the antibiotic resistance gene-free miniplasmid according to any one of claims 1-9 or the recombinant host cell according to claim 10, and an optionally pharmaceutically acceptable excipient.

15. A system for producing a plasmid, comprising a host cell and a plasmid, wherein the host cell comprises a nucleotide sequence encoding a toxin protein, and the plasmid comprises a nucleotide sequence encoding an antitoxin protein;
an amino acid sequence of the toxin protein is shown in SEQ ID NO: 4, and an amino acid sequence of the antitoxin protein comprises: an amino acid sequence shown in SEQ ID NO: 1, or an amino acid sequence having one or more mutations of E24D, I36V and V43I compared with SEQ ID NO: 1; or
an amino acid sequence of the toxin protein is shown in SEQ ID NO: 13, and an amino acid sequence of the antitoxin protein comprises: an amino acid sequence shown in SEQ ID NO: 9, or an amino acid sequence having one or more mutations of T6I, T43A, K47E, A50S, E51D, G52A and N54K compared with SEQ ID NO: 9.

16. The system according to claim 15, wherein the amino acid sequence of the toxin protein is shown in SEQ ID NO: 4, and the amino acid sequence of the antitoxin protein is shown in SEQ ID NO: 1, SEQ ID NO: 2, or SEQ ID NO: 3; or the amino acid sequence of the antitoxin protein is shown in SEQ ID NO: 13, and the amino acid sequence of the antitoxin protein is shown in SEQ ID NO: 9, SEQ ID NO: 10, SEQ ID NO: 11 or SEQ ID NO: 12.

17. The system according to claim 15 or 16, wherein the host cell is a Gram-negative bacterium, preferably *Escherichia coli,* such as an *Escherichia coli* GT115 strain, a Top10 strain, a DH5a strain, or a BL21 (DE3) strain.

18. The system according to any one of claims 15-17, wherein the host cell comprises a gene expression cassette for inducibly expressing the toxin, the gene expression cassette for the toxin protein comprises an operon or a promoter for inducibly expressing the toxin protein, and preferably, the operon or the promoter is selected from a Lac lactose operon, an L-arabinose inducible pBAD promoter, an L-rhamnose inducible rhapBAD promoter, or a λPL/PR-clts857 thermosensitive promoter.

19. The system according to any one of claims 15-18, wherein the plasmid comprises a replicon, and preferably, the replicon is selected from ColE1, ColE2, pMB1, pSC101, RSF, R6K, pUC57, RK2, and p15A, preferably R6K or pUC57.

20. The system according to any one of claims 15-19, wherein the plasmid does not comprise an antibiotic resistance gene, and/or a length of a plasmid backbone is ≤ 1,000 bp, preferably ≤ 900 bp, ≤ 800 bp or ≤ 600 bp.

21. The system according to claim 19 or 20, the replicon and/or the nucleotide sequence encoding an antitoxin protein do not comprise a CpG motif.

22. A method for producing a plasmid by using the system according to any one of claims 15-21, comprising the following steps:
(1) transforming the plasmid comprising the nucleotide sequence encoding an antitoxin protein into the host cell comprising the nucleotide sequence encoding a toxin protein;
(2) inducibly expressing the toxin protein during culture of a bacterial host cell and expressing the antitoxin protein; and
(3) obtaining a plasmid,
wherein preferably, the plasmid is the antibiotic resistance gene-free miniplasmid according to any one of claims 1-9.

23. A method for producing a target recombinant peptide, polypeptide or protein by using the system according to any one of claims 15-21, wherein the plasmid further comprises a gene expression cassette encoding the target recombinant peptide, polypeptide or protein, and the method comprises culturing the system and recovering the recombinant peptide, polypeptide or protein of interest.

24. A genetically engineered bacterium, wherein a cell of the genetically engineered bacterium comprises a nucleotide sequence encoding a toxin protein, an amino acid sequence of the toxin protein is shown in SEQ ID NO: 4 or 13, and preferably, the nucleotide sequence encoding a toxin protein is within a genome of the genetically engineered bacterium.

25. The genetically engineered bacterium according to claim 24, wherein the genetically engineered bacterium further comprises a plasmid, and the plasmid comprises a nucleotide sequence encoding an antitoxin protein;
an amino acid sequence of the toxin protein is shown in SEQ ID NO: 4, and an amino acid sequence of the antitoxin protein comprises: an amino acid sequence shown in SEQ ID NO: 1, or an amino acid sequence having one or more mutations of E24D, I36V and V43I compared with SEQ ID NO: 1; or
an amino acid sequence of the toxin protein is shown in SEQ ID NO: 13, and an amino acid sequence of the antitoxin protein comprises: an amino acid sequence shown in SEQ ID NO: 9, or an amino acid sequence having one or more mutations of T6I, T43A, K47E, A50S, E51D, G52A and N54K compared with SEQ ID NO: 9; and
preferably, the plasmid is the antibiotic resistance gene-free miniplasmid according to any one of claims 1-9.

26. The genetically engineered bacterium according to claim 24 or 25, wherein the genetically engineered bacterium is a Gram-negative bacterium, preferably *Escherichia coli,* such as an *Escherichia coli* GT115 strain, a Top10 strain, a DH5a strain, or a BL21 (DE3) strain.

27. A kit, comprising the antibiotic resistance gene-free miniplasmid according to any one of claims 1-9, the recombinant host cell according to claim 10, the system according to any one of claims 15-21, or the genetically engineered bacterium according to any one of claims 24-26.

28. Use of the antibiotic resistance gene-free miniplasmid according to any one of claims 1-9, the recombinant host cell according to claim 10, the system according to any one of claims 15-21, the genetically engineered bacterium according to any one of claims 24-26, or the kit according to claim 27 in preparing a drug for gene therapy, a drug for cell therapy and a DNA vaccine, and producing a virus or an antibody.

29. Use of the antibiotic resistance gene-free miniplasmid according to any one of claims 1-9, the recombinant host cell according to claim 10, the system according to any one of claims 15-21, the genetically engineered bacterium according to any one of claims 24-26, or the kit according to claim 27 in maintaining stability of a plasmid in a bacterial host cell.
